# EUROPEAN PATENT SPECIFICATION

(11) **EP 1 788 977 B1**
(45) Date of publication and mention of the grant of the patent: **19.03.2008**
(21) Application number: 05795415.8
(22) Date of filing: 01.09.2005
(51) Int. Cl.: A61F 2/06

(54) **MODULAR PROSTHESIS AND METHOD FOR BRANCH VESSELS**
MODULARE PROTHESE UND VERFAHREN FÜR VERZWEIGTE GEFÄSSE
PROTHESE MODULAIRE ET PROCEDE DE CONNEXION DE VAISSEAUX

(30) Priority: 02.09.2004 US 607238 P; 02.09.2004 US 607184 P
(43) Date of publication of application: 30.05.2007
(73) Proprietor: MED INSTITUTE, INC., West Lafayette Indiana 47906 (US)
(72) Inventor: FEARNOT, Neal, E., West Lafayette, IN 47906 (US)
(74) Representative: Ellis, Katherine Elizabeth Sleigh
(86) International application number: PCT/US2005/031202
(87) International publication number: WO 2006/028925

(56) References cited:
- WO-A-20/04110314
- WO-A-20/05034808
- US-A1- 2003 088 306
- US-A1- 2004 193 254
- US-B1- 6 814 752

## Description

### TECHNICAL FIELD

This invention relates to medical devices and more particularly, to endoluminal devices suitable for various medical applications and the methods for making and using such endoluminal devices.

### BACKGROUND

Throughout this specification, when discussing the application of this invention to the aorta or other blood vessels, the term "distal" with respect to an abdominal device is intended to refer to a location that is, or a portion of the device that when implanted is, further downstream with respect to blood flow; the term "distally" means in the direction of blood flow or further downstream. The term "proximal" is intended to refer to a location that is, or a portion of the device that when implanted is, further upstream with respect to blood flow; the term "proximally" means in the direction opposite to the direction of blood flow or further upstream.

The functional vessels of human and animal bodies, such as blood vessels and ducts, occasionally weaken or even rupture. For example, the aortic wall can weaken, resulting in an aneurysm. Upon further exposure to hemodynamic forces, such an aneurysm can rupture. In Western European and Australian men who are between 60 and 75 years of age, aortic aneurysms greater than 29 mm in diameter are found in 6.9% of the population, and those greater than 40 mm are present in 1.8% of the population.

One intervention for weakened, aneurismal, dissected or ruptured vessels is the use of an endoluminal device or prosthesis such as a stent graft to provide some or all of the functionality of the original, healthy vessel and/or preserve any remaining vascular integrity by replacing a length of the existing vessel wall that contains the site of vessel weakness or failure. Stent grafts for endoluminal deployment are generally formed from a tube of a biocompatible material in combination with one or more stents to maintain a lumen therethrough. Stent grafts effectively exclude the defect by sealing both proximally and distally to the defect, and shunting blood through its length. A device of this type can, for example, treat various arterial aneurysms, including those in the thoracic aorta or abdominal aorta.

The bifurcated stent graft, one example of an endoluminal prosthesis, is known for use in treating abdominal aortic aneurysms, where the stent graft at the proximal end defines a single lumen for placement within the aorta and at the other end bifurcates into the iliac arteries. One such stent graft, disclosed in PCT application WO98/53761, is useful for repair of abdominal aortic aneurysms. That application discloses a stent graft that includes a sleeve or tube of biocompatible graft material such as woven polyester fabric or polytetrafluoroethylene (PTFE) defining a main lumen and two iliac limbs. The stent graft further includes several stents secured therealong. The stent graft is designed to span an aneurysm that extends along the aorta between the iliac and renal arteries.

In the WO98/53761 application, the fabric-covered portion of the single-lumen proximal end of the stent graft bears against the wall of the aorta above the aneurysm and distal to the renal arteries to seal off the aneurysm. Thin wire struts of a juxtarenal attachment stent traverse the renal artery ostia without occluding them. Barbs on the attachment stent help anchor the stent graft in place.

An extension module may be attached to one of the limbs of the stent graft to extend through a respective iliac artery and, optionally, an iliac extension module can be connected to the other leg. The deployment of a modular stent graft into the lumen of a patient from a remote location by the use of an introducer or introducer assembly is disclosed in the same patent application.

One stent graft approved by the Food and Drug Administration (FDA) to treat aortic aneurysms is the ZENITH^{®} AAA Endovascular Graft (Cook Incorporated, Bloomington, Indiana). The ZENITH^{®} AAA Endovascular Graft is made up of three prosthetic modules: a bifurcated main body module and two leg modules. The main body is positioned in the aorta. The legs are positioned in the iliac arteries and connect to the main body. The stent graft thus extends from a section of the aorta, typically below the renal arteries and into both iliac arteries. The graft material is made of a woven polyester fabric like that used in open surgical repair. Standard surgical suturing techniques are used to sew the graft material to a frame of stainless steel stents. These self-expanding stents provide support for the graft material.

US 2003/088 306 discloses a modular endoluminal prosthesis with a first module comprising a trunk and a branch and a second module comprising a trunk and a notch, the notch and the branch being alignable.

An endoluminal prosthesis may be comprised of multiple prosthetic modules. A modular prosthesis allows a surgeon to accommodate a wide variation in vessel morphology while reducing the necessary inventory of differently sized prostheses. For example, aortas vary in length, diameter and angulation between the renal artery region and the region of the aortic bifurcation. Prosthetic modules that fit each of these variables can be assembled to form a prosthesis, obviating the need for a custom prosthesis or large inventories of prostheses that accommodate all possible combinations of these variables. A modular system may also accommodate deployment options by allowing the proper placement of one module before the implantation of an adjoining module.

Modular prostheses are typically assembled in situ by overlapping the tubular ends of the prosthetic modules so that the end of one module sits partially inside the other module, preferably forming circumferential apposition through the overlap region. This attachment process is called "telescoping." The connections between prosthetic modules are typically maintained by the friction forces at the overlap region and enhanced by the radial force exerted by the internal prosthetic module on the external prosthetic modules where the two overlap. The fit may be further enhanced by stents attached to the modules at the overlap region.

A length of a vessel which may be treated by these prostheses may have one or more side branch vessels. The celiac, superior mesenteric, left carotid and renal arteries, for example, are side branch vessels of the aorta. If these side branch vessels are blocked by the prosthesis, the original blood circulation is impeded, and the patient can suffer. If, for example, the celiac artery is blocked by the prosthesis, the patient can experience abdominal pain, weight loss, nausea, bloating and loose stools associated with mesenteric ischemia. The blockage of any branch vessel may be associated with unpleasant or even life-threatening symptoms.

When treating a vessel with an endoluminal prosthesis, it is therefore preferable to preserve the original circulation by providing a prosthetic branch that extends from the prosthesis to a side branch vessel so that the blood flow into the branch vessel is not impeded. For example, the aortic section of the ZENITH^{®} abdominal aortic stent graft (Cook Incorporated, Bloomington, Indiana), described above, can be designed to extend above the renal arteries and to have prosthetic side branches that extend into the renal arteries. Branch extension prosthetic modules ("branch extensions") can form a telescoping connection to the prosthetic branch to complete the prosthesis. Furthermore, some aneurysms extend into the branch vessels in both the thoracic and abdominal aorta. Deploying prostheses with prosthetic branches into these vessels may help prevent expansion and/or rupture of these aneurysms. High morbidity and mortality rates are associated with these aneurysms.

### BRIEF SUMMARY

As an example to understand the invention, there is a method of deploying a modular endoluminal prosthesis in a body lumen that includes providing a first prosthetic module that has a first notch and a first prosthetic side branch that is integral with a first prosthetic trunk, and deploying the first prosthetic module so that the first prosthetic side branch is inserted into a first side branch vessel. The branch and the notch may have a particular orientation relative to one another. The method may further comprise aligning the first notch with a second side branch vessel.

The method may further comprise providing a second prosthetic module comprising a second notch and/or a second integral branch and deploying the second prosthetic module so that a telescoping interconnection is established between the first prosthetic module and the second prosthetic module; either the second notch may be aligned with the first side branch vessel or the second integral branch may be inserted into a second side branch vessel. The second prosthetic module may also have both an integral side branch and a second notch; in this case the method may further include deploying the second prosthetic module so that the second notch aligns with the first side branch vessel. The first and second prosthetic modules may be deployed so that the first overlaps the second or so that the second overlaps the first. The second prosthetic module may also comprise a third notch which may align with a third side branch vessel.

The method may further comprise providing and deploying a third prosthetic module so that it a telescoping interconnection is established between the third prosthetic module and the second prosthetic module. The third prosthetic module may include a third notch which may be aligned with the second side branch vessel. The third prosthetic module may also comprise a third prosthetic side branch. The second prosthetic module may further comprise a third notch and the method may include deploying the second prosthetic module so that the third notch aligns with a third side branch vessel. Any of the first, second or third notches may be a fenestration.

As another example useful to understand the invention, there is a method of connecting endoluminal prosthetic modules in a body lumen that includes providing a first prosthetic module, wherein the first prosthetic module comprises a first prosthetic trunk integral with a first prosthetic side branch. The first prosthetic module is deployed so that the first prosthetic side branch is inserted into a first side branch of the body lumen. The body lumen may be an aorta. A second prosthetic module includes a second prosthetic trunk integral with a second prosthetic side branch and is deployed so that the second prosthetic module is in fluid communication with the first prosthetic module and so that the second prosthetic side branch is inserted into a second side branch of the body lumen. The method may include deploying an intervening prosthetic module between the first prosthetic module and the second prosthetic module to establish a telescoping interconnection between the intervening prosthetic module and both the first and second prosthetic modules, or a telescoping interconnection may be formed directly between the first prosthetic module and the second prosthetic module. The method may also include deploying a third prosthetic module, wherein the third prosthetic module comprises a third prosthetic trunk in fluid communication with and integral with a third prosthetic side branch. The third prosthetic module may be deployed so that it is in fluid communication with the second prosthetic module and so that the third prosthetic side branch is inserted into a third side branch of the body lumen. A telescoping interconnection may also be established between the first prosthetic module and the second prosthetic module and/or between the second prosthetic module and the third prosthetic module and/or between the first prosthetic module and the third prosthetic module.

The method may further comprise reducing the diameter of the first prosthetic side branch by using at least one tie and deploying the first prosthetic module by inserting the first prosthetic side branch into the first branch vessel and releasing the at least one tie. This may be accomplished in part by deploying the second prosthetic side branch though the aorta. A tie may also be used to reduce the diameter of the second prosthetic side branch during deployment. The first prosthetic side branch and/or the second prosthetic side branch may be deployed into a left subclavian artery, a left carotid artery, an innominate artery, a renal artery, a celiac artery, or a superior mesenteric artery. The first prosthetic module or the second prosthetic module may be deployed through one of the innominate artery, the left subclavian artery or the left carotid artery.

The method may further comprise deploying the first prosthetic trunk while gripping at least a portion of the first prosthetic branch; seating the first prosthetic module by retracting the first prosthetic branch; and deploying the first prosthetic branch. This method of deployment may also be used for the second prosthetic module. The first prosthetic trunk may define a first notch, which may be aligned with the second prosthetic branch. The second prosthetic trunk may define a second notch, which may be aligned with the first prosthetic branch; the first notch may also be aligned with the second prosthetic branch.

The first prosthetic module may be deployed through one of the either the aorta or the first side branch and the second prosthetic module may deployed through the other of the aorta or the first side branch. Both the first prosthetic module and the second prosthetic module may also be deployed through the aorta. The first prosthetic module may also be deployed though the first side branch vessel and the second prosthetic module though the second side branch vessel.

As part of the invention, there is a modular endoluminal prosthesis for deployment in a body lumen that includes a first prosthetic module with a first prosthetic side branch integral with a first prosthetic trunk, and a first notch in the first prosthetic trunk. The first notch may have a circumferential orientation with respect to the first prosthetic side branch. The first prosthetic module may have at least one stent, which may be asymmetrical. The first notch may be a fenestration. The first notch and the first prosthetic side branch may be generally on opposite sides or on the same side of the first prosthetic trunk.

The prosthesis includes a second prosthetic module, wherein a telescoping interconnection is established when the first and the second prosthetic modules are interconnected. The first prosthetic module overlaps the second prosthetic module or the second prosthetic module overlaps the first prosthetic module. The second prosthetic module may include a second notch and/or a second prosthetic side branch integral with a second prosthetic trunk. The second notch may be aligned with the first prosthetic side branch when the first and the second prosthetic modules are interconnected. Also, the second notch and the second prosthetic side branch may be on opposite sides of the second prosthetic trunk, such that, when interconnected, the first and the second prosthetic side branches extend in opposite directions.

The second notch and the second prosthetic side branch may be on a single side of the second prosthetic trunk, such that, when interconnected, the first and the second prosthetic side branches extend in a similar direction. The first notch and the second prosthetic side branch may be aligned when the first and the second prosthetic modules are interconnected. The second notch may also straddle the first prosthetic side branch. The prosthesis may also include a third prosthetic module, wherein a telescoping interconnection is established when the second and the third prosthetic modules are interconnected.

In yet another aspect of the invention, there is a modular endoluminal prosthesis for deployment in a body lumen that includes a first prosthetic module, wherein the first prosthetic module comprises a first prosthetic trunk integral with a first prosthetic side branch; and a second prosthetic module, wherein the second prosthetic module comprises a second prosthetic trunk integral with a second prosthetic side branch. A telescoping interconnection is established when the first and the second prosthetic modules are interconnected. When interconnected, the first prosthetic module may overlap the second prosthetic module or the second prosthetic module may overlap the first prosthetic module. The first prosthetic trunk may include a first notch. The first notch and the first prosthetic side branch may be on a single side of the first prosthetic trunk such that, when interconnected, the first and the second prosthetic side branches extend in generally the same direction from the device. Also, the first notch and the first prosthetic side branch may be on opposite sides of the first prosthetic trunk such that, when interconnected, the first and the second prosthetic side branches extend in generally opposite directions from the device.

The first or second prosthetic module may comprise a fenestration. The prosthesis may also include a third prosthetic module that, when interconnected, forms a telescoping interconnection with the second prosthetic module. The second notch and the third prosthetic side branch may be aligned. The third prosthetic module may have a third prosthetic side branch. At least one stent may be affixed to the first, second and/or third prosthetic modules. The stent may be an asymmetrical zigzag stent designed to avoid obstructing a notch or a prosthetic side branch. A nitinol reinforcing wire may be attached to a margin of the notch of the first or second prosthetic module.

In yet another aspect of the invention there is a modular endoluminal prosthesis for deployment in a body lumen that comprises a first prosthetic module comprising a first prosthetic side branch integral with a first prosthetic trunk. The prosthesis also comprises a first notch in the first prosthetic trunk, wherein the first notch has a circumferential orientation with respect to the first prosthetic side branch.

In yet another aspect of the invention, there is a modular endoluminal prosthesis for deployment in a body lumen that comprises a first prosthetic module, wherein the first prosthetic module comprises a first prosthetic trunk integral with a first prosthetic side branch. The prosthesis further comprises a second prosthetic module, wherein the second prosthetic module comprises a second prosthetic trunk having a first notch. A telescoping interconnection is established when the first and the second prosthetic modules are interconnected.

In yet another aspect of the invention, there is a modular endoluminal prosthesis for deployment in a body lumen that comprises a first prosthetic module, wherein the first prosthetic module comprises a first prosthetic trunk integral with a first prosthetic side branch. The prosthesis further comprises a second prosthetic module, wherein the second prosthetic module comprises a second prosthetic trunk integral with a second prosthetic side branch. A telescoping interconnection is established when the first and the second prosthetic modules are interconnected.

### BRIEF DESCRIPTION OF THE DRAWINGS

Figures 1a-f show six different embodiments of a prosthetic module;

Figures 1g-h show two different embodiments of a notch;

Figure 2 shows a composite prosthesis implanted in the aortic arch and thoraco-abdominal aorta;

Figure 3 shows a composite prosthesis implanted in the abdominal aorta;

Figure 4 shows the interconnection between two prosthetic modules, one of which has an integral prosthetic branch;

Figure 5 shows one embodiment of an interconnection between two prosthetic modules, each having an integral prosthetic branch;

Figure 6 shows another embodiment of an interconnection between two prosthetic modules, each having an integral prosthetic branch;

Figure 7 shows the interconnection between three prosthetic modules, each having an integral prosthetic branch, and deployed into a schematically presented aortic arch;

Figure 8 shows a composite endoluminal prosthesis which is adapted for deployment into the abdominal and thoraco-abdominal aorta;

Figures 9a-c show the deployment of a prosthetic module into the aortic arch;

Figures 10a-c show the deployment of a prosthetic module to form a telescoping interconnection with the prosthetic module of Figures 9a-c;

Figure 11 shows an embodiment of an introducer;

Figure 12 shows an alternative embodiment of an introducer;

Figure 13 shows the proximal end of the introducer according to either Figure 11 or Figure 12;

Figure 14 shows a longitudinal cross-sectional view of the proximal end introducer shown in Figure 13 and showing one embodiment of prosthetic module retained therein;

Figure 15 shows a transverse cross-sectional view of a prosthetic side branch of a prosthetic module, including a diameter-reducing tie arrangement;

Figures 16-19 show four additional embodiments of a prosthetic module;

Figure 20 shows a schematic view of the first stage of deployment of a prosthetic module into an aortic arch of a patient using the introducer of Figure 11 or Figure 12 so that the module forms a telescoping interconnection with an already deployed module;

Figure 21 shows a further stage in the deployment process commenced in Figure 20 with the auxiliary guide wire snared;

Figure 22 shows a further stage in the deployment process commenced in Figure 20 with a protective catheter passed over auxiliary guide wire;

Figure 23 shows a further stage in the deployment process commenced in Figure 20 with the sheath on the introducer withdrawn partially;

Figure 24 shows a further stage in the deployment process commenced in Figure 20 with the prosthetic side branch drawn into the left subclavian artery;

Figure 25 shows the first stage of release of the prosthetic module from the introducer in the deployment process commenced in Figure 20;

Figure 26 shows the second stage of release of the prosthetic module from the introducer in the deployment process commenced in Figure 20;

Figure 27 shows the final stage of release of the prosthetic module from the introducer in the deployment process commenced in Figure 20;

Figure 28 shows a detailed view of the prosthesis shown in Figure 27 with a modified left subclavian branch;

Figure 29 shows a detailed view of another embodiment of the left subclavian branch shown in Figure 27;

Figure 30 shows a detailed view of another embodiment of the left subclavian branch shown in Figure 27;

Figure 31 shows a general external view of another embodiment of the introducer;

Figure 32 shows a longitudinal cross-sectional view of the introducer shown in Figure 31;

Figure 33 shows the introducer of Figure 32 but after withdrawal of the sheath;

Figure 34 shows the introducer of Figure 33 but after activation of the sliding handle;

Figure 35 shows a detailed longitudinal cross-sectional view of the sliding and fixed handle portion of one embodiment of a introducer;

Figure 36 shows a view of the embodiment shown in Figure 35 after withdrawal of the capsule;

Figure 37 shows a side view of the embodiment shown in Figure 35;

Figure 38 shows a side view of the embodiment shown in Figure 36 after withdrawal of the capsule;

Figure 39 shows a general view of an embodiment of an introducer with a prosthetic module partially released;

Figure 40 shows a partly cross-sectional view of the embodiment shown in Figure 39;

Figure 41 shows another embodiment of a prosthetic module;

Figure 42 shows a cross-sectional view of the prosthetic module of Figure 41;

Figure 43 shows yet another embodiment of a prosthetic module;

Figure 44 shows a cross-sectional view of the prosthetic module shown in Figure 43;

Figure 45 shows yet another embodiment of a prosthetic module;

Figure 46 shows a cross-sectional view of the prosthetic module shown in Figure 45;

Figure 47 shows a prosthetic module flexed to fit into a section of the aortic arch;

Figure 48 shows a cross-sectional view of the proximal end of a prosthetic module showing a sealing stent;

Figure 49 shows further detail of the fastening shown in Figure 48;

Figures 50a and 50b show prosthetic modules having outwardly extending barbs;

Figures 51a and 51b show prosthetic modules having inwardly extending barbs;

Figure 52a and 52b show zones on prosthetic modules suitable for the placement of elements that impede migration and slippage;

Figures 53a, 53b and 53c show the operation of mutually engageable cuffs; and

Figures 54a and 54b show the deployment of prosthetic modules through a band of warp fibers.

### DETAILED DESCRIPTION

To help understand this description, the following definitions are provided.

The term "prosthesis" means any replacement for a body part or function of that body part. It can also mean a device that enhances or adds functionality to a physiological system.

The term "endoluminal" describes objects that are found or can be placed inside a lumen in the human or animal body. A lumen can be an existing lumen or a lumen created by surgical intervention. This includes lumens such as blood vessels, parts of the gastrointestinal tract, ducts such as bile ducts, parts of the respiratory system, etc. An "endoluminal prosthesis" is thus a prosthesis that can be placed inside one of these lumens. A stent graft is a type of endoluminal prosthesis.

The term "stent" means any device or structure that adds rigidity, expansion force or support to a prosthesis. A zigzag stent is a stent that has alternating struts and peaks (i.e., bends) and defines a generally cylindrical space. A "Gianturco Z stent" is a type of self-expanding zigzag stent

The term "side branch vessel" refers to a vessel that branches off from a side of a main vessel. Thus, it should be seen that "side branch vessel" and "main vessel" are relative terms. The "side branch vessels" of the thoracic and abdominal aorta include the celiac, inferior phrenic, superior mesenteric, lumbar, inferior mesenteric, middle sacral, middle suprarenal, renal, internal spermatic, ovarian (in the female), innominate, left carotid, and left subclavian arteries. The iliac arteries are not side branch arteries.

The term "prosthetic trunk" refers to a portion of a prosthesis that shunts blood through a main vessel. A "trunk lumen" runs through the prosthetic trunk.

The term "prosthetic side branch" refers to a portion of a prosthesis that is anastomosed to the prosthetic trunk and shunts blood into and/or through a side branch vessel. An integral prosthetic side branch is one that has been connected to the trunk or formed with the trunk before deployment within the body.

"Anastomosis" refers to any existing or established connection between two lumens, such as the prosthetic trunk and prosthetic branch, that puts the two in fluid communication with each other. An anastomosis is not limited to a surgical connection between blood vessels, and includes a connection between a prosthetic branch and a prosthetic trunk that are formed integrally.

The term "branch extension" refers to a prosthetic module that can be deployed within a branch vessel and connected to a prosthetic branch.

The term "pull-out force" means the maximum force of resistance to partial or full dislocation provided by a modular prosthesis. The pull-out force of a prosthesis having two interconnected modules may be measured by an MTS ALLIANCE RT/5^{®} tensile testing machine (MTS Corporation, Eden Prairie, Minnesota). The MTS machine is connected to a computer terminal that is used to control the machine, collect, and process the data. A pressurization pump system is attached to the load cell located on the tensile arm of the MTS machine. One end of the prosthesis is connected to the pressurization pump, which provides an internal pressure of 60mm Hg to simulate the radial pressure exerted by blood upon the device when deployed in vivo. The other end of the prosthesis is sealed. The prosthesis is completely immersed in a 37°C water bath during the testing to simulate mean human body temperature. The MTS machine pulls the devices at 0.1mm increments until the devices are completely separated. The computer will record, *inter alia,* the highest force with which the modules resist separation, i.e. the pull-out force.

An endoluminal prosthesis may be assembled in situ from multiple prosthetic modules. In order to prevent the occlusion of a side branch vessel, it is preferable to provide a prosthetic side branch extending from one or more of the prosthetic modules in order to preserve flow to those side branch vessels. These prosthetic side branches are preferably integral with the prosthetic trunk of the prosthetic module.

Figures la-f show external views of six embodiments of a prosthetic module 2. The prosthetic module includes a tubular body that forms the prosthetic trunk 3 and may have a tubular body connected to the trunk 3 through an anastomosis that forms the prosthetic side branch 4. Both the side branch 4 and trunk 3 are formed from a biocompatible woven or non-woven fabric or other graft material.

Biocompatible fabrics, non-woven materials and porous sheets may be used as the graft material. Examples of biocompatible polymers from which porous sheets can be formed include polyesters, such as poly(ethylene terephthalate), polylactide, polyglycolide and copolymers thereof; fluorinated polymers, such as PTFE, expanded PTFE and poly(vinylidene fluoride); polysiloxanes, including polydimethyl siloxane; and polyurethanes, including polyetherurethanes, polyurethane ureas, polyetherurethane ureas, polyurethanes containing carbonate linkages and polyurethanes containing siloxane segments. In addition, materials that are not inherently biocompatible may be subjected to surface modifications in order to render the materials biocompatible. Examples of surface modifications include graft polymerization of biocompatible polymers from the material surface, coating of the surface with a crosslinked biocompatible polymer, chemical modification with biocompatible functional groups, and immobilization of a compatibilizing agent such as heparin or other substances. Thus, any polymer that may be formed into a porous sheet can be used to make a graft material, provided the final porous material is biocompatible. Polymers that can be formed into a porous sheet include polyolefins, polyacrylonitrile, nylons, polyaramids and polysulfones, in addition to polyesters, fluorinated polymers, polysiloxanes and polyurethanes as listed above. Preferably the porous sheet is made of one or more polymers that do not require treatment or modification to be biocompatible.

The graft material may include a biocompatible polyurethane. Examples of biocompatible polyurethanes include THORALON^{®} (Thoratec, Pleasanton, CA), BIOSPAN^{®}, BIONATE^{®}, ELASTHANE^{™}, PURSIL^{™} and CARBOSIL^{™} (Polymer Technology Group, Berkeley, CA). As described in U.S. Patent Application Publication No. 2002/0065552 A1, THORALON^{®} is a polyetherurethane urea blended with a siloxane-containing surface modifying additive. Specifically, the polymer is a mixture of base polymer BPS-215 and an additive SMA-300.

The graft material may also include extracellular matrix materials. The "extracellular matrix" is a collagen-rich substance that is found in between cells in animal tissue and serves as a structural element in tissues. It is typically a complex mixture of polysaccharides and proteins secreted by cells. The extracellular matrix can be isolated and treated in a variety of ways. Following isolation and treatment, it is referred to as an "extracellular matrix material," or ECMM. ECMMs may be isolated from submucosa (including small intestine submucosa), stomach submucosa, urinary bladder submucosa, tissue mucosa, renal capsule, dura mater, liver basement membrane, pericardium or other tissues.

Purified tela submucosa, a preferred type of ECMM, has been previously described in U.S. Patent Nos. 6,206,931, 6,358,284 and 6,666,892 as a bio-compatible, non-thrombogenic material that enhances the repair of damaged or diseased host tissues. Purified submucosa extracted from the small intestine ("small intestine submucosa" or "SIS") is a more preferred type of ECMM for use in this invention. Another type of ECMM, isolated from liver basement membrane, is described in U.S. Patent No. 6,379,710. ECMM may also be isolated from pericardium, as described in U.S. Patent No. 4,502,159.

The prosthetic trunk has two ends 5, 7, one of which is proximal and the other distal, depending on the orientation in situ. The prosthetic trunk may have a diameter in the range of about 10 mm to about 60 mm and a length from about 50 mm to 500 mm, depending on the size of the vasculature. The prosthetic trunk 3 may be tapered, outwardly bulging like a balloon or of constant diameter along its length depending upon the topography of the vasculature.

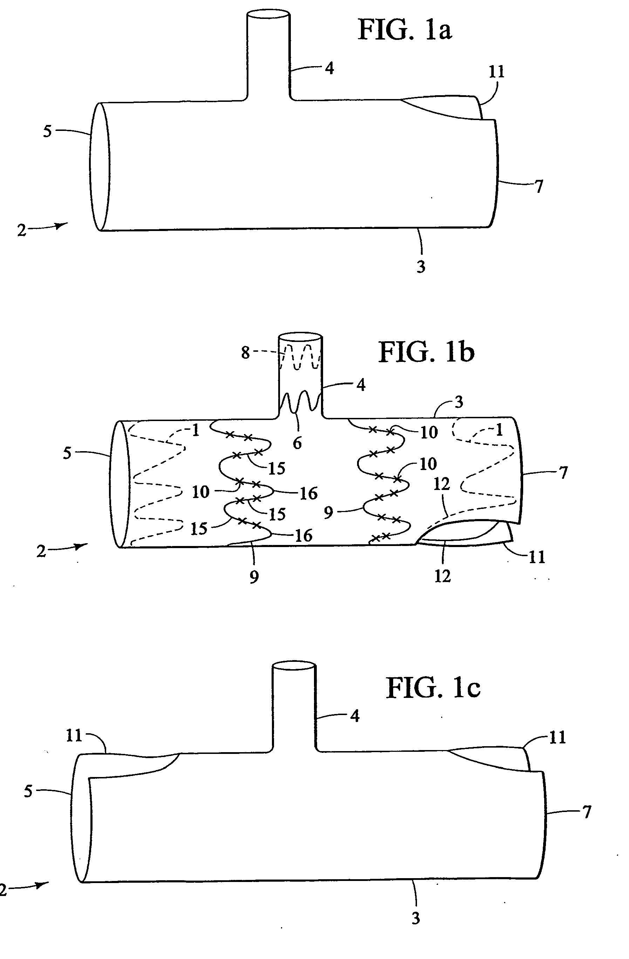

Along the length of the prosthetic trunk, there may be a number of self-expanding zigzag stents 9 such as the Gianturco Z stent on the outside of the body, as shown in Figure 1b. In this embodiment there are two external stents 9.

At one or both ends 5, 7 of the prosthetic module 2 there may be an internal zigzag stent 1 which helps seal against a vascular wall or an interconnecting module. The outer surface of the tubular body 3 at the ends 5, 7 presents an essentially smooth outer surface that can engage and seal against the wall of the aorta or an adjoining prosthetic module when it is deployed. The stents 1, 9 are comprised of struts 15 with bends 16 at each end of the struts 15. Barbs may extend distally from the struts of the stents through the graft material to engage the surrounding vessel wall to prevent distal movement of the prosthesis that may be caused by pulsatile blood flow through the prosthesis. The stents 1, 9 are joined to the graft material by means of stitching 10, preferably using a monofilament or braided suture material.

The prosthetic modules of Figures 1a-e have an integral prosthetic branch 4 extending from the prosthetic trunk 3. The prosthetic side branch 4 may be formed from a separate piece of graft material and attached to a fenestration in the prosthetic trunk 3 using sutures or other attachment means known to those of skill in the art. Alternatively, the prosthetic trunk 3 and prosthetic side branch 4 could be formed as a single unit. The prosthetic side branch 4 is preferably of a size and shape suitable for the branch vessel in which it is to be deployed.

Stents may be attached to the prosthetic side branch 4, in the manner described above in reference to the prosthetic trunk. The prosthetic side branch 4 preferably has a distal self-expanding zigzag stent 8 attached internally and a proximal self-expanding zigzag stent 6 attached externally. The prosthetic branch 4 may instead have only a single self-expanding zigzag stent attached to its distal end.

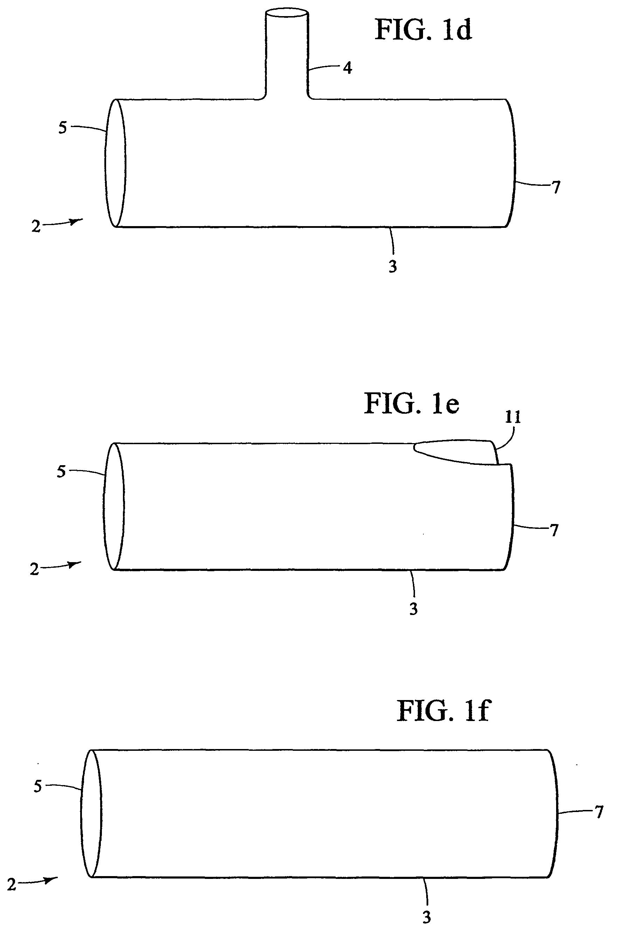

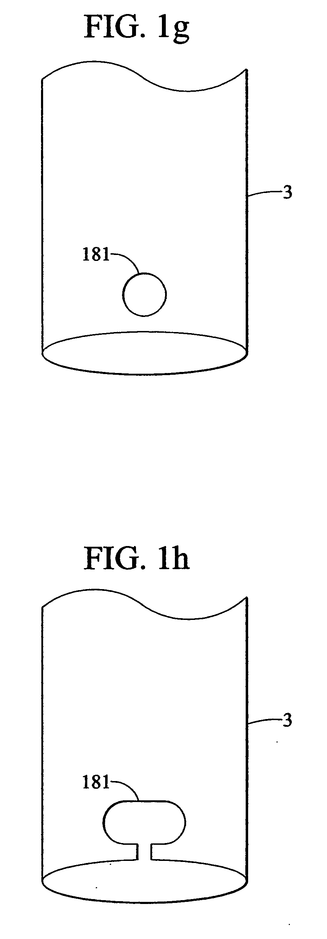

The prosthetic trunks 3 of Figures 1a-c and 1e have notches 11 at one or both ends. The notches 11 are sized and shaped either so that they do not occlude a prosthetic branch of an externally telescoped module, or so that they straddle a prosthetic branch of an internally telescoped module, as described in further detail below, although they may be larger in any dimension than necessary for those particular tasks, to allow a greater range of deployment options. A notch, by definition, includes both scallops and fenestrations. Two additional embodiments of a notch are shown in Figures 1g-h. Figure 1g shows a notch 181 that is a fenestration. Figure 1h shows a notch 181 that has a keyhole shape for increasing the range of radial orientation relative to an interconnecting module or branch vessel anastomosis. The notch can be fully or partially elliptical, circular or oblong.

The notch 11 may be supported by a segment of a self-expanding zigzag stent segment that terminates on both sides of the notch 11. The notch 11 may also be supported by a self-expanding zigzag stent that has an amplitude such that it does not obstruct the notch 11. Likewise, a self-expanding zigzag stent may have a lengthened pair of adjoining struts 12 that define the notch 11, as shown in Figure 1b. The notch may also be supported by a wire, preferably a nitinol wire, sewn to the margin of the notched end of the prosthetic module 2.

The prosthetic module 2 of Figure 1a has a single notch 11 and a single prosthetic branch 4 on the same side; such a prosthetic module 2 may, for example, be used in the celiac artery, superior mesenteric artery ("SMA"), or one of the thoracic branches, as described below. The prosthetic module 2 of Figure 1b has a single notch 11 and a single prosthetic branch 4 located on opposite sides; such a prosthetic module 2 may, for example, be used in a renal artery. The prosthetic module 2 of Figure 1c has two notches 11 and a single prosthetic branch 4, all on the same side; such a prosthetic module may, for example, be used in a left carotid artery. The prosthetic module 2 of Figure 1d has a single prosthetic branch 4, and may be deployed at any intersection of a branch vessel and a vascular trunk. The prosthetic module 2 of Figure 1e has a single notch 11 in one of its ends, and may, for example, be used in a left subclavian artery. Figure 1f has neither a trunk nor a notch, and is, for example, suitable for connecting some of the modules described above.

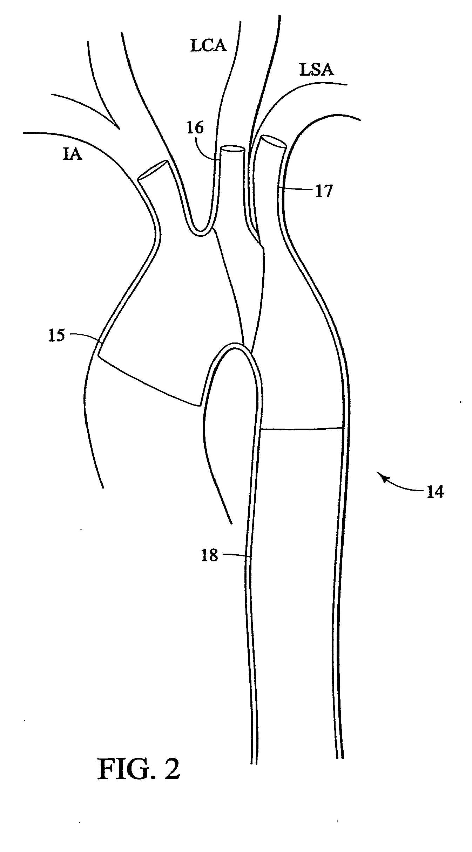

The modules 2 described above may be used, for example, to fully or partially graft the aortic arch. The branch modules shown in Figures 1a-d may be employed to ensure that there is adequate blood flow to the branch vessels of that region, including the innominate artery ("IA"), the left carotid artery ("LCA"), and the left subclavian artery ("LSA"). A composite prosthesis 14 shown in Figure 2 is deployed into the three main thoracic side branches. Separate modules 15, 16, 17 are used for each of the IA, LCA and LSA, respectively. An additional prosthetic module 18 extends into the thoraco-abdominal aorta and the abdominal aorta.

Such an endoluminal prosthesis 14 does not necessarily extend proximally to the IA. For example, the modules 16, 17 associated with the LCA or the LSA 16, 17 may be the most proximal of the prosthetic modules. If the LCA is the most proximally stent grafted branch vessel of the aortic arch, it may be preferable to have that prosthetic module extend further in a proximal direction, but provide an additional fenestration or notch to ensure that the IA is not occluded. Likewise, if the LSA is the most proximally stent grafted branch vessel of the aortic arch, it may be preferable to have that prosthetic module 17 extend further in a proximal direction but provide an additional fenestration or notch to ensure that LCA, or even the IA, are not occluded.

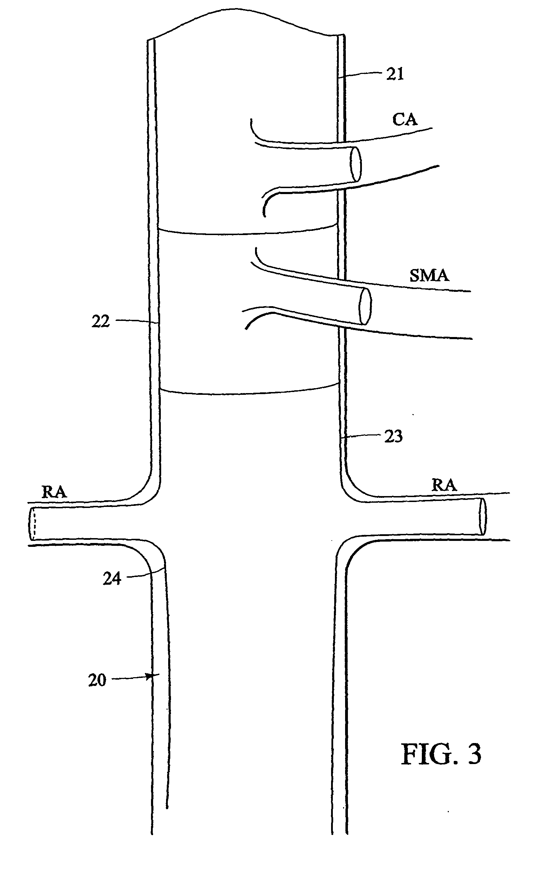

The modules described above may be used to fully or partially graft the thoraco-abdominal aorta and the abdominal aorta. The branch modules shown in Figures 1a-1d may be employed to ensure that there is adequate blood flow to the branch vessels of that region, including the celiac artery ("CA"), the SMA or the renal arteries ("RA"). A composite prosthesis 20 shown in Figure 3 has all four main abdominal and thoraco-abdominal side branches grafted. A separate module 21-24 is used for each of the right RA, left RA, CA and SMA.

Such an endoluminal prosthesis 20 does not necessarily extend proximally to the CA. For example, the module associated with the SMA 22 or the RAs 23, 24 may be the most proximal of the prosthetic modules. If the SMA is the most proximally grafted branch vessel of the aorta, it may be preferable to have that prosthetic module 22 extend further in a proximal direction but provide an additional fenestration or notch to ensure that the CA is not occluded. Likewise, if the right and left RAs are the most proximally stent grafted branch vessels of the abdominal aorta, it may be preferable to have those prosthetic modules 23, 24 extend further in a proximal direction but provide an additional fenestration or notch to ensure that SMA, or even the CA, are not occluded.

Such an endoluminal prosthesis 20 does not necessarily extend distally to the RAs. For example, the module 22 associated with the SMA may be the most distally grafted side branch. If so, it may be preferable to have that module 22 extend distally to the RAs but provide notches or fenestration so the RAs are not occluded.

The entire prostheses 14, 20 shown in Figures 2 and 3, or portions of those prostheses may be connected to form a prosthesis that extends to both the thoracic and abdominal aorta.

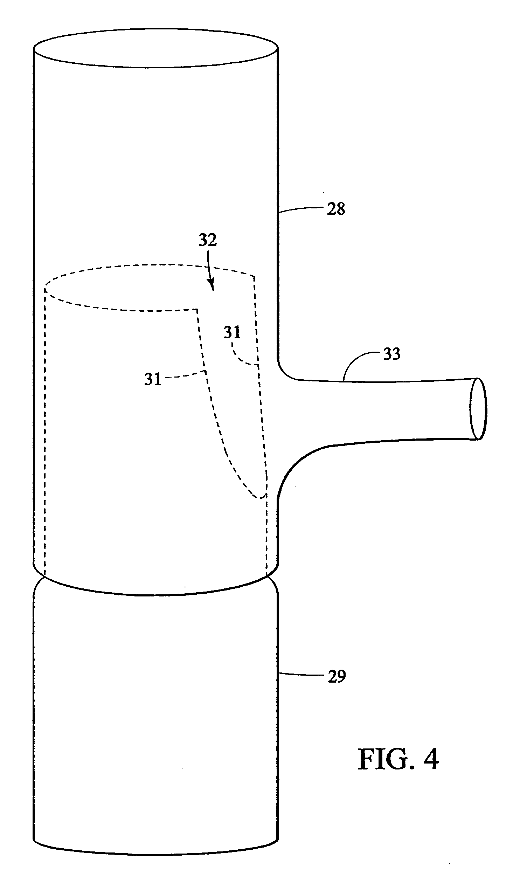

Figure 4 shows the telescoping connections between two prosthetic modules 28, 29. The notched module 29, when deployed, may be placed distally or proximally with respect to the branched module 28. As shown, the notched module 29 is inside the branched module 28, but it is also possible to have the modules interconnected so the notched module 29 is on the outside, such that the notch 32 is aligned with and straddles the branch 33 and the sides 31 of the notch 32 abut the branch 33. The notch 32 is aligned with the branch 33. The branched module 28 may also have an additional notch in one or both of its ends. When it is said that a notch is aligned with a prosthetic branch, that means that it straddles the prosthetic branch if the notch is on the external module, or is placed so as not to obstruct the prosthetic branch ostium if the notch is on the internal module. When a notch is aligned with a branch vessel, that means that it is placed so as not to obstruct the branch vessel ostium.

Figure 4 demonstrates that the notch-branch combination may permit a greater overlap between two interconnected modules 28, 29, thus increasing the pull-out force. Figure 4 demonstrates that for each integral side branch vessel part, there is preferably a corresponding notch on the other prosthetic module to increase the overlap. This design can be particularly useful for closely spaced side branch vessels, such as the LCA and LSA, or opposing side branch vessels, such as the two RAs, as described below. For example, the left carotid artery and left subclavian artery branch may be spaced by even less than 0.5 cm at their origins. Because of the proximity of the branch vessels in certain parts of the aortic anatomy, conventional modular interconnections may not have sufficient overlap to maintain adequate pull-out forces. The interconnection between the modules may be further supported with glue, barbs or other elements, as described below.

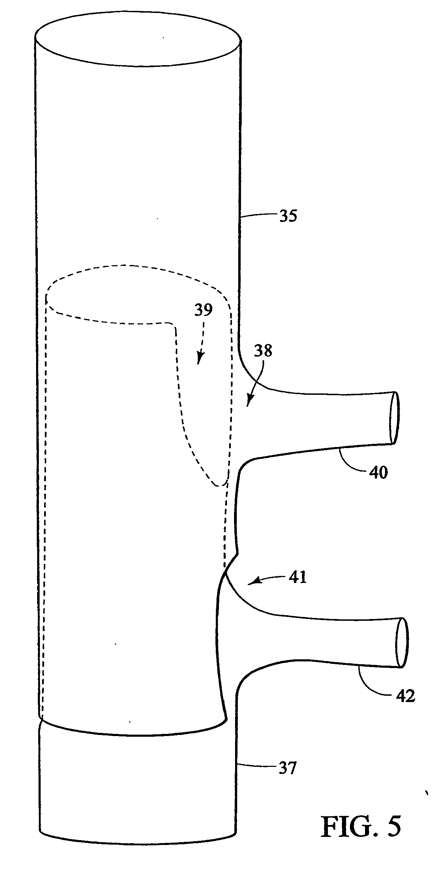

Figure 5 shows an interconnection between two modules 35, 37 suitable for grafting two side-by-side branch vessels, such as the CA and SMA. Both modules 35, 37 are similar to the module shown in Figure 1a. As shown in Figure 5, the notch 39 of the second module 37 aligns with the anastomosis 38 of the branch 40 of the other module 35 so that the prosthetic branch 40 of the first module 35 is not obstructed. The notch 41 of the first module 37 preferably straddles the prosthetic branch 42 of the second prosthetic module 37, as shown.

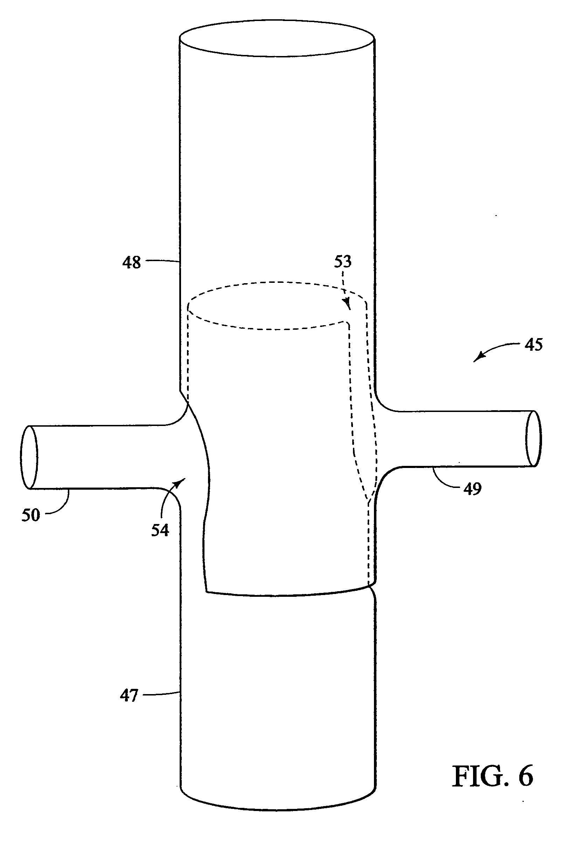

Figure 6 shows a modular interconnection 45 suitable for grafting both renal arteries or other similarly situated vessels. The modules 47, 48 in Figure 6 are similar to those described in relation to Figure 1b, each module 47, 48 having a branch 49, 50 and notch 53, 54 on opposite sides. As shown, the first prosthetic branch 49 and the second prosthetic branch 50 are at about 180° to each other. However, the interconnection shown may be modified to vary the angle between the prosthetic branches to better suit a particular vascular topography. As shown, the first and second prosthetic banks 49, 50 are at the same relative vertical position on the prosthesis. However, the interconnection shown may be modified to vary the relative vertical position of the prosthetic branches 49, 50 to better suit a particular vascular topography. To make these variations there is preferably a corresponding change in the positions of the notches 53, 54.

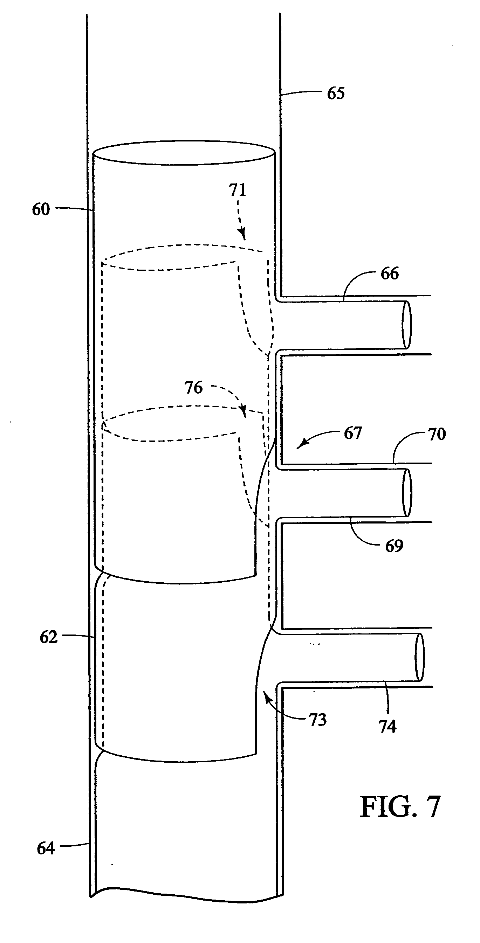

Figure 7 shows a schematic depiction of three branched prosthetic modules 60, 62, 64 deployed in the aortic arch 65. The arch 65 is depicted schematically, without curvature, to better display the interaction between the prosthetic modules 60, 62, 64. The first module 60, which in this case is the most proximal module, is similar to the prosthetic module of Figure 1a. The prosthetic branch 66 extends into the IA. The distal notch 67 of the first prosthetic module 60 does not obstruct the LCA, and straddles the neighboring branch 69.

A second prosthetic module 62 that is similar to the prosthetic module of Figure 1e may be deployed into the first prosthetic module 60. The second prosthetic module 62 is preferably deployed into the aorta so that the prosthetic branch 69 extends into the SMA 70. The proximal notch 71 of the second prosthetic module 62 is positioned so that the prosthetic branch 66 of the first module 60 is not obstructed. The second prosthetic module 62, as shown, has a distal notch 73 that straddles the prosthetic branch 74 of the third module 64.

Within the second prosthetic module 62 is deployed a third prosthetic module 64 that is also similar to the prosthetic module of Figure 1a. The third prosthetic module 64 is preferably deployed into the aorta so that its prosthetic branch 74 extends into the LSA. The proximal notch 76 of the third prosthetic module 64 is positioned so that the prosthetic branch 69 of the second module 62 is not obstructed.

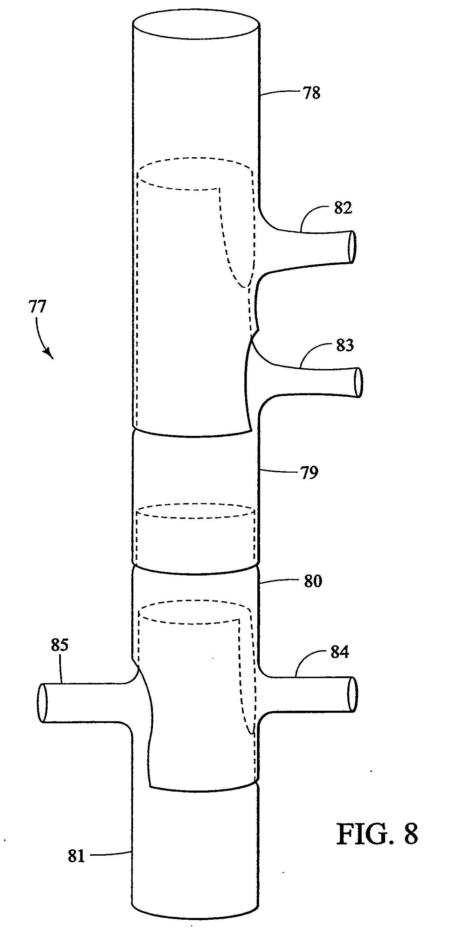

Figure 8 shows a composite prosthesis 77 that may be suitable for deployment into an aorta and providing blood flow into the CA, SMA and RAs. Four prosthetic modules 78-81 make up the prosthesis, each with an integral prosthetic branch 82-85.

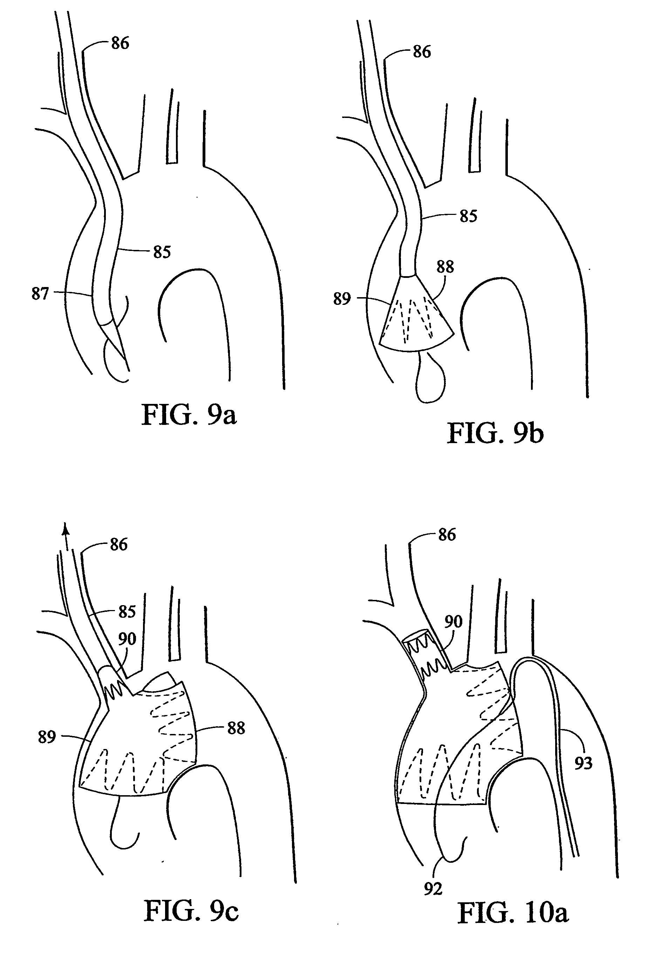

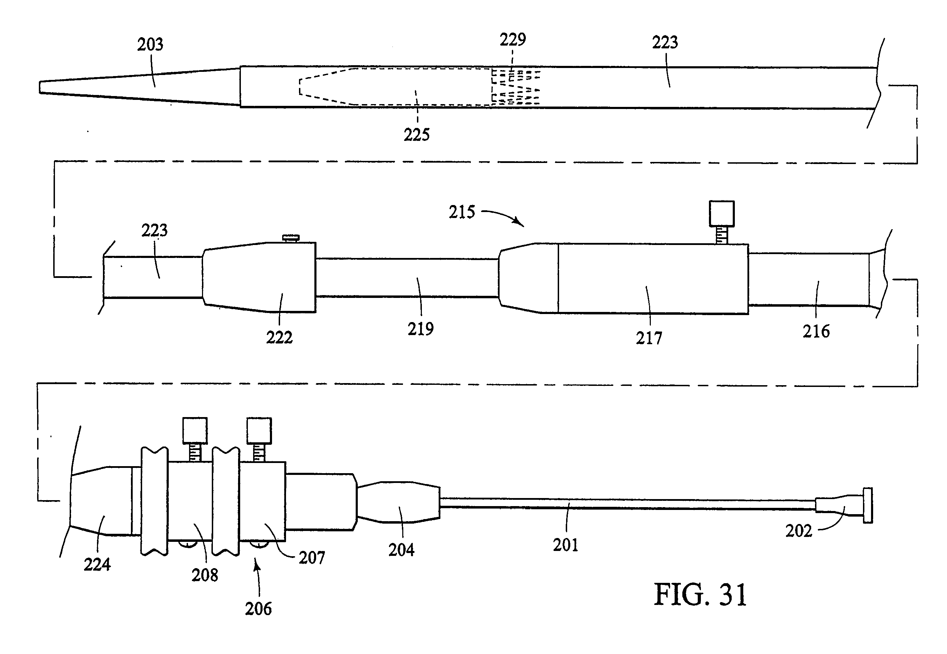

A prosthetic module that has an integral prosthetic side branch may be deployed through a side branch artery. As shown in Figures 9a-c, a thoracic module may be deployed through the IA, into the aorta. To do so, the introducer 85 is inserted through a cut-down 86 in the right common carotid, through the IA, and inserted into the aorta. The introducer 85 may be a sheath known to those of skill in the art, or a more complex introducer, as described with respect to Figures 31-40. When the introducer 85 reaches a preselected location within the artery (Figure 9a), the sheath 87 is retracted, exposing the aortic section 89 of the prosthetic module 88 (Figure 9b). The aortic section 89 of the module 88 is deployed while the prosthetic branch section 90 is gripped by the introducer (Figure 9c). As shown in Figure 9c, the prosthetic branch section 90 is gripped by retaining that portion within the sheath. The introducer 85 is then retracted so that the module 88 is seated in the vessel, after which the prosthetic branch 90 is deployed. "Seating" means that the introducer is manipulated in order to get a prosthetic module to better position itself within the vasculature. Seating the module may be facilitated by gripping a portion of the module after another portion is deployed, so that the module can be moved.

The method of Figures 9a-c may be used to deploy a prosthetic module though any branch vessel, including, for example, the LSA and the LCA. This method may also be used to deploy a module to form a telescoping interconnection with a previously deployed prosthetic module. For example, the LCA module 95 shown in Figures 10a-c could be deployed through the LCA, and a third branched module (not shown) may be deployed through the left subclavian artery. When this method is used to deploy a telescoping module, the method may help establish a suitable overlap between the modules and help accommodate the respective angulation and vertical positions of neighboring branch arteries.

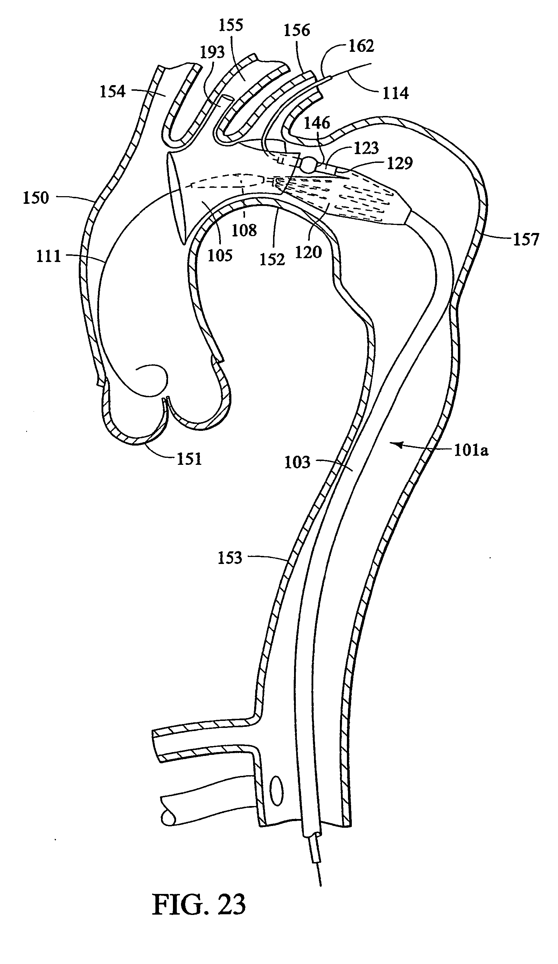

A prosthetic module may be deployed through a branch vessel with the assistance of diameter-reducing ties (not shown) around the aortic sections of the prosthetic module. Similar ties are discussed with reference to Figures 23-27. These may prevent the prosthetic trunk from engaging the aortic wall or previously deployed modules prematurely. After the prosthetic trunk has been deployed and the module has been seated, the diameter-reducing ties may be released.

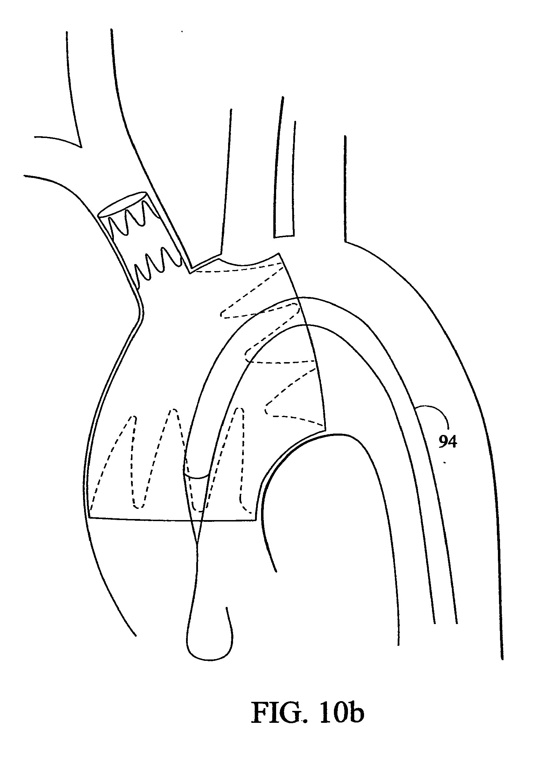

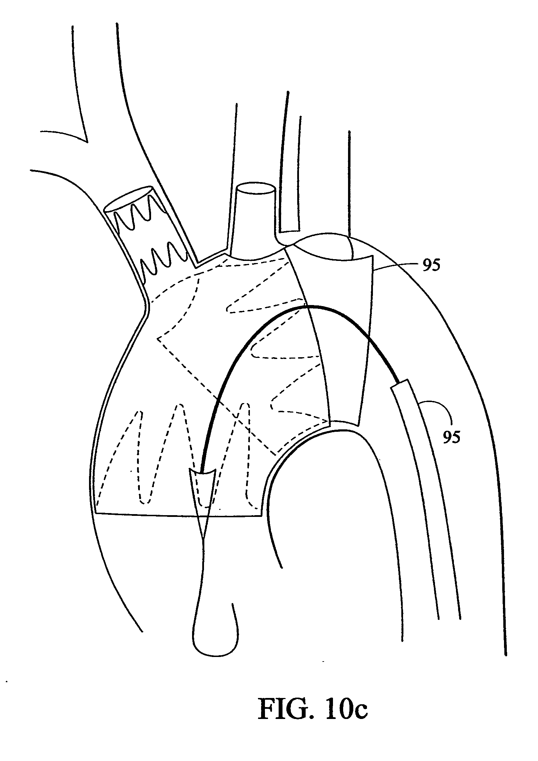

As shown in Figures 10a-c, branched modules may also be deployed through a trunk vessel, such as the aorta. A femoral cut down is the preferred entry point for the introducer, although the introducer may be inserted from other vessels. Figures 10a-c depict the deployment of a prosthetic module 95 that forms a telescoping interconnection with the prosthetic module 88 of Figures 9a-c, to form the interconnection shown in Figure 5. In order to form an adequate overlap between the modules, the first module is preferably seated and deployed, as described above, before the introduction of a telescoping module 95. A guide wire 92 is first inserted using a catheter 93. Then the introducer 94 is inserted to a predetermined location (Figure 10b). The sheath is then retracted releasing the second prosthetic module into the LCA and aortic arch (Figure 10c). The deployment steps of Figures 10a-c may be repeated for an additional branched module (not shown) for the left subclavian artery. Deployment methods are further described in U.S. Patent Application Serial No. 10/653,401, filed September 2, 2003 and U.S. Patent Application Serial No. 60/510,244, filed October 10, 2003. Deployment through the aorta is described in greater detail below.

It is important to note that the deployment methodology described above may be adapted so that the distal prosthetic modules are deployed prior to the proximal prosthetic modules. Thus, a proximal module would be deployed internally to the interconnecting distal module. This may, in certain situations, prevent the occurrence of a wind-sock effect, whereby blood flow can "catch" an inner, distal module.

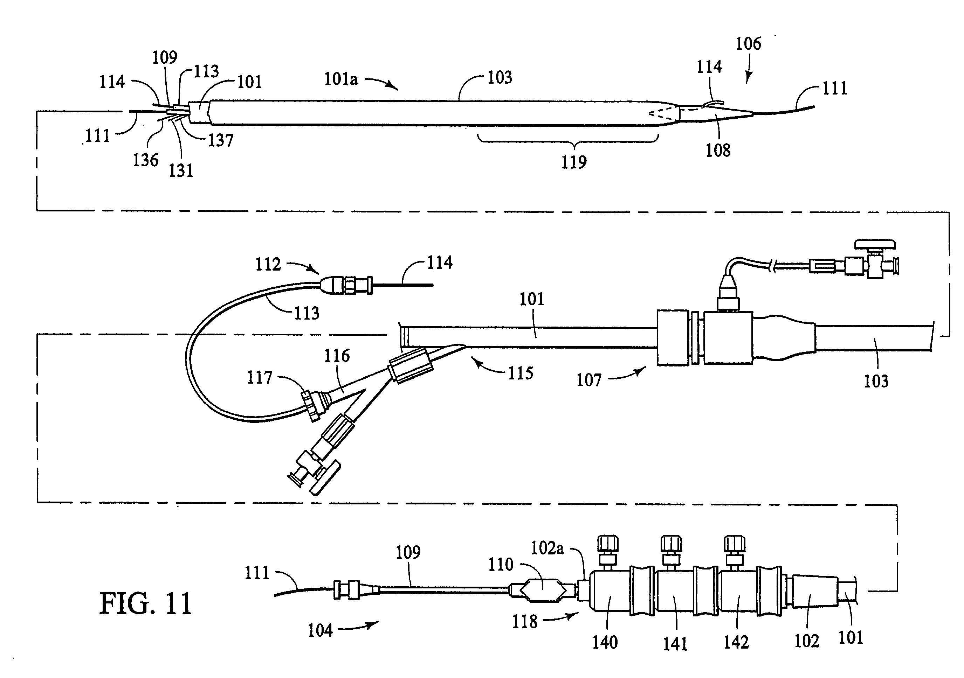

Figures 11 to 15 show preferred embodiments of introducers for deployment through the aorta; the introducers may be modified for deployment through a side branch. In each case the same reference numerals will be used for corresponding components or parts. It will be seen that the introducer 101a comprises a deployment catheter 101 with a handle 102 at the distal end generally shown as 104. Covering a portion of the deployment catheter 101 is a sheath 103 extending proximally from a sheath manipulator 107.

At the proximal end 106 of the introducer 101a is a nose cone 108. The nose cone is fastened to the guide wire catheter 109 which extends from the distal end 104 of the introducer to the nose cone. A guide wire 111 extends through the guide wire catheter 109. A pin vice 110 locks the guide wire catheter with respect to the deployment catheter 101 at the distal end 102a of the handle 102.

In the embodiment shown in Figure 11, proximally of the handle 102 is a "Y" piece 115 in the deployment catheter 101 with a side arm 116 extending from the "Y" piece. Extending through the side arm 116 and through a seal 117 is an auxiliary catheter 113 with an auxiliary guide wire 114 through the auxiliary catheter 113. A grip and syringe adaptor 112 at the distal end of the auxiliary catheter 113 enables connection of a syringe to flush the auxiliary catheter 113 as required.

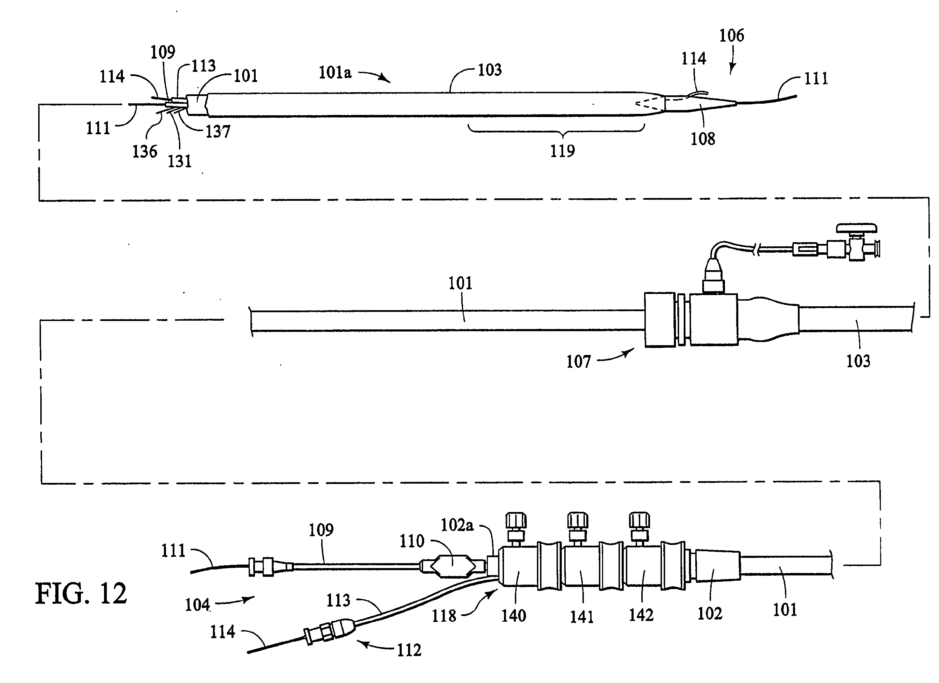

In the embodiment shown in Figure 12, an auxiliary catheter 113 with an auxiliary guide wire 114 extending through the auxiliary catheter 113 extends from the distal end 102a of the handle 102. A grip and syringe adaptor 112 at the distal end of the auxiliary catheter 113 enables connection of a syringe to flush the auxiliary catheter 113 as required. On the handle 102 is mounted a set of trigger wire release arrangements generally shown as 118 which will be discussed in detail later.

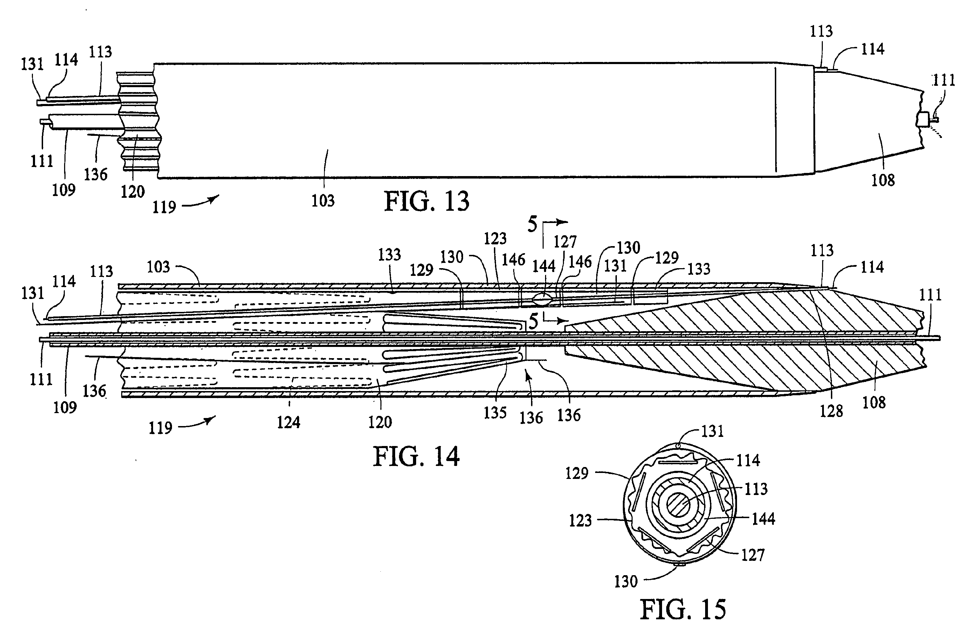

It will be noted that the nose cone 108 has, as particularly shown in Figure 14, a longitudinal notch 128 through which passes the auxiliary catheter 113 and auxiliary guide wire 114 so that it extends just beyond the sheath 103. This means that once the introducer has been deployed in substantially the correct position the auxiliary guide wire 114 can be advanced beyond the nose cone 108 so that it can be snared from the side branch artery as will be discussed later.

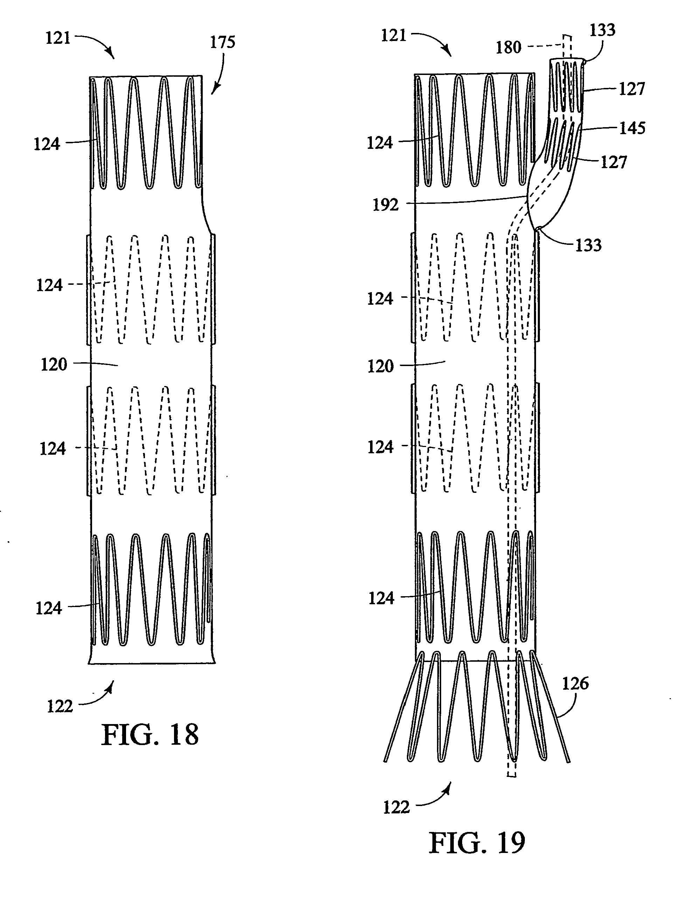

The embodiment of a prosthetic module 120 shown in Figure 19 is partially shown in a compressed condition on the introducer in Figure 14. Extending substantially laterally from the tubular body 120 and nearer the proximal end 121 is a prosthetic side branch 123 again with a lumen through it which is continuous with the lumen of the tubular body 120. As can be particularly seen in Figure 14, when the prosthetic module 120 is in a compressed state within the sheath 103, the prosthetic side branch 123 is directed proximally.

As seen in Figure 19, the prosthetic module 120 has zigzag style Gianturco Z stents 124 along its length with a distally extending uncovered zigzag style Gianturco Z stent 126. It should be noted that in some embodiments of the prosthetic module for placement in the aortic arch the distally extending uncovered zigzag style Gianturco Z stent 126 may not be present. The prosthetic side branch 123 also has zigzag Gianturco Z stents 127. The stents are inside the prosthetic module 120 at the proximal and distal ends and outside the prosthetic module 120 between the proximal and distal ends. The number of zigzag Gianturco Z stents along the length of the tubular graft 120 will depend upon the length of the prosthetic module 120.

As can be seen in Figure 14 and in detail in Figure 15, the prosthetic side branch 123 is held in a diameter reduced condition for deployment by means of diameter reducing ties 129. The diameter reducing ties 129 are lengths of suture material which are fastened to the graft material at 130 and are looped around a trigger wire 131 on the opposite side of the prosthetic side branch and pulled tight so that the diameter of the prosthetic side branch is reduced. When the trigger wire 131 is released as will be discussed later, the loops of the diameter reducing ties are released and the prosthetic side branch can expand to its full size. After release the diameter reducing ties remain fixed to the graft material of the prosthetic side branch.

The auxiliary catheter includes a bulge or "acorn" 144 where it passes through the prosthetic side branch 123 with extra diameter reducing ties 146 either side of it. These diameter reducing ties 146 effectively grip the auxiliary guide wire catheter either side of the bulge or "acorn." By this arrangement the auxiliary catheter cannot be moved with respect to the prosthetic side branch unless the diameter reducing ties 146 are removed. This facilitates the moving of the prosthetic side branch by preventing relative movement of the prosthetic side branch with respect to the auxiliary catheter 112. The extra diameter reducing ties 146 can be released by the same trigger wire 131 that is used to release the diameter reducing ties 129. Also on the prosthetic side branch 123 are radio-opaque markers 133 which enable the position of the prosthetic side branch to be observed by suitable radio-graphic techniques.

The proximal end 121 of the prosthetic module 120 is retained in a compressed condition and attached to the guide wire catheter 109 by a release arrangement 135 and a trigger wire 136 to release the release arrangement 135 is also present. Such a retention and release arrangement is depicted in PCT Publication WO 2004/017868.

A further trigger wire release arrangement (not shown in Figure 14) is used to retain the distal end of the prosthetic module at the proximal end of the deployment catheter 101. This distal release arrangement may include a capsule for the exposed stent 126 (see Figure 19). Such a capsule system is depicted in PCT Publication WO 98/53716. The use of a distal capsule system and a deployment system to release an exposed stent is described in U.S. Provisional Patent Application Serial No. 60/392,667, filed June 28, 2002.

It will be noted that the auxiliary catheter 113 and the auxiliary guide wire 114 pass through the lumen of the prosthetic module 120 as well as the lumen of the prosthetic side branch 123 and then out through the notch 128 in the nose cone 108.

The trigger wire release arrangements 118 on the handle 102 include three trigger wire release mechanisms. A first trigger wire release mechanism 140 is used to release the distal prosthetic module release mechanism via trigger wire 137, a second trigger wire release mechanism 141 is used to release the proximal end prosthetic module release mechanism 135 via trigger wire 136 and the third trigger wire release mechanism 142 is used to pull the trigger wire 131 which releases the diameter reducing ties 129. The trigger wire release mechanisms are operated in the order discussed as will be explained also later with respect to the various stages of deployment according to one embodiment of the invention. In some embodiments of the invention there may be only two trigger wire release mechanisms such as where the proximal and distal ends of the prosthetic module are retained by the same trigger wire.

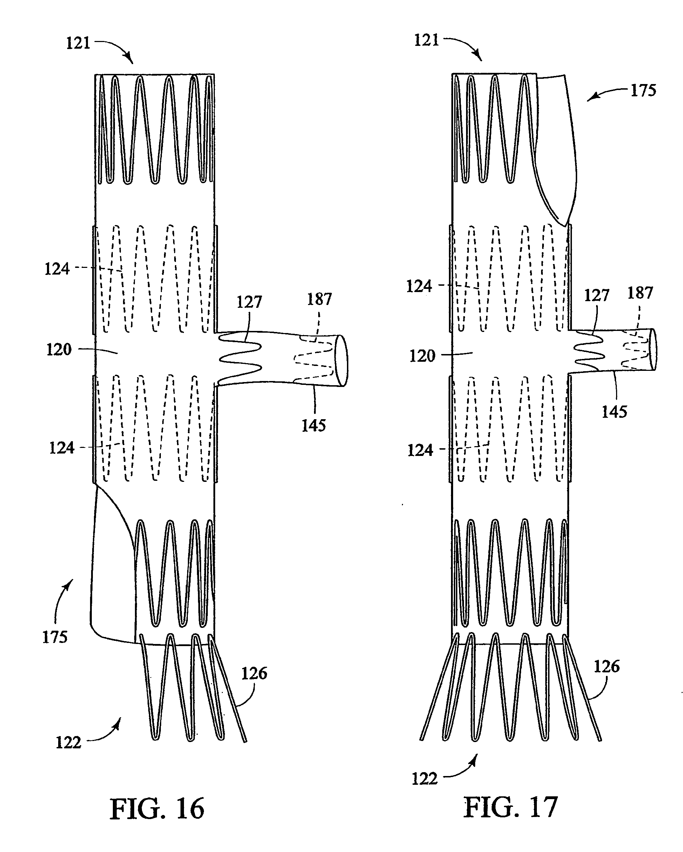

Various embodiments of prosthetic module suitable for use with the present invention will now be discussed with reference to Figure 16 to 19. In each case the same reference numerals will be used for corresponding components or parts. Each of the prosthetic modules 120 has a tubular body of a biocompatible graft material and zigzag style Gianturco Z stents 124 along its length. Figures 16, 17 and 19 show a distally extending uncovered zigzag style Gianturco Z stent 126, although in Figure 16, the stent is truncated to accommodate the notch 175. In some embodiments of the prosthetic module, the distally extending uncovered zigzag style Gianturco Z stent 126 may not be present; this may be preferable for deployment into the aortic arch, especially when the prosthetic module 120 is not the most distally located module. The use of a composite stent grafting system is further described in PCT publication WO 2004/017867.

The stents are inside the prosthetic module 120 at the proximal and distal ends 121, 122 and outside the prosthetic module 120 between the proximal end 121 and the distal end 122. The number of zigzag Gianturco Z stents along the length of the tubular graft 120 will depend upon the length of the prosthetic module 120. Normally the zigzag Gianturco Z stents are spaced apart to allow a degree of flexibility of the prosthetic module so that it can more easily fit the shape of a vessel into which it is deployed. In each case the prosthetic module includes a fenestration but the treatment of the fenestration varies.

The prosthetic branches 145 shown in Figures 16, 17 and 19 are stented. In Figures 16 and 17 there is shown an internal distal stent 187 and an external proximal stent 127. The prosthetic branch 145 of Figure 19 has two external stents 127. The notches 175 can be of varying size, shape and orientation relative to the branch 145.

In Figure 19, a fenestration 192 has a prosthetic branch 145 sewn to it. The fenestration 192 may also have a reinforcement ring of nitinol or similar resilient wire around its periphery. The branch 145 includes one or more external zigzag Gianturco Z stents 127. The dotted line 180 shows how the auxiliary guide wire catheter passes through the branch 145. The prosthetic side branch 123 includes radio-opaque markers 133 to assist with placement.

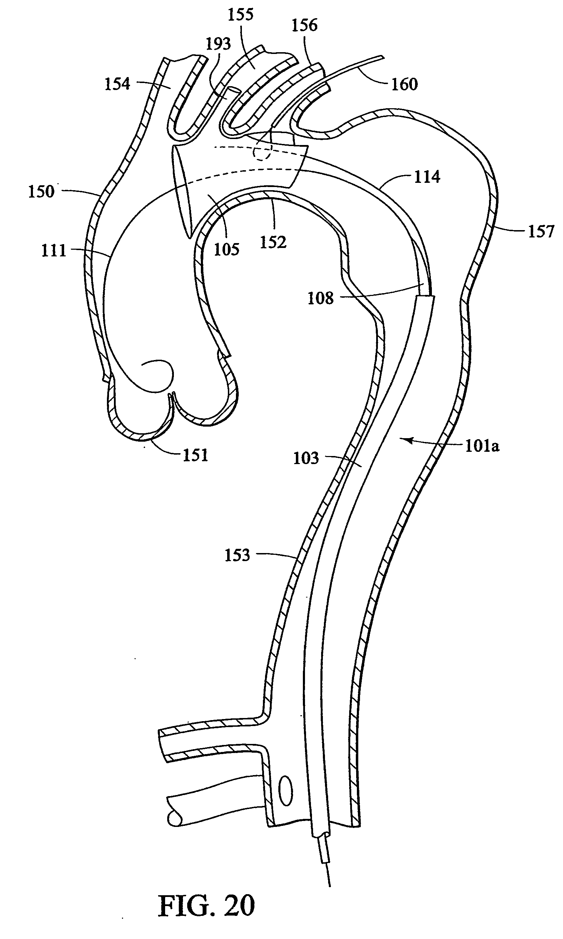

The various stages of deployment of one embodiment of the prosthetic module 120 into the aortic arch of a patient will now be discussed with reference to Figures 20 to 27. The introducer can be the type shown in either Figure 11 or Figure 12.

The aortic arch region of a patient generally comprises an ascending aorta 150 extending from an aortic valve 151 of the heart of the patient, then over the aortic arch 152 to the descending aorta 153. From the aortic arch three main arteries extend. These are the innominate artery 154, the left carotid artery 155 and a left subclavian artery 156. This embodiment will generally be discussed with reference to deployment of a prosthetic module with a prosthetic side branch into the aorta and left subclavian artery but the invention is not so restricted. The prosthetic module is deployed so that it forms a telescoping interconnection with another prosthetic module 105, as shown in Figures 20-27. As the most proximal module 105 in this prosthesis, fixation to the aorta may be enhances with barbs or hooks (not shown) that extend proximally from the proximal edge of this module 105. These hooks or barbs would engage the vascular tissue, thereby helping prevent distal migration as a result of the flow and pulsatile forces in the aorta. Similarly, loops that merge and become trapped by the inner surface of the vessel could also contribute to enhanced fixation.

An endoluminal prosthesis may be necessary in the aortic arch region when an aneurysm 157 in the aorta extends up the aorta to such an extent that there is insufficient patent aortic wall to provide good sealing for a endoluminal prosthesis distally of the left subclavian artery 156. It is desirable in such circumstances to extend the endoluminal prosthesis to seal onto good artery wall at least between the left carotid artery 155 and the left subclavian artery 156.

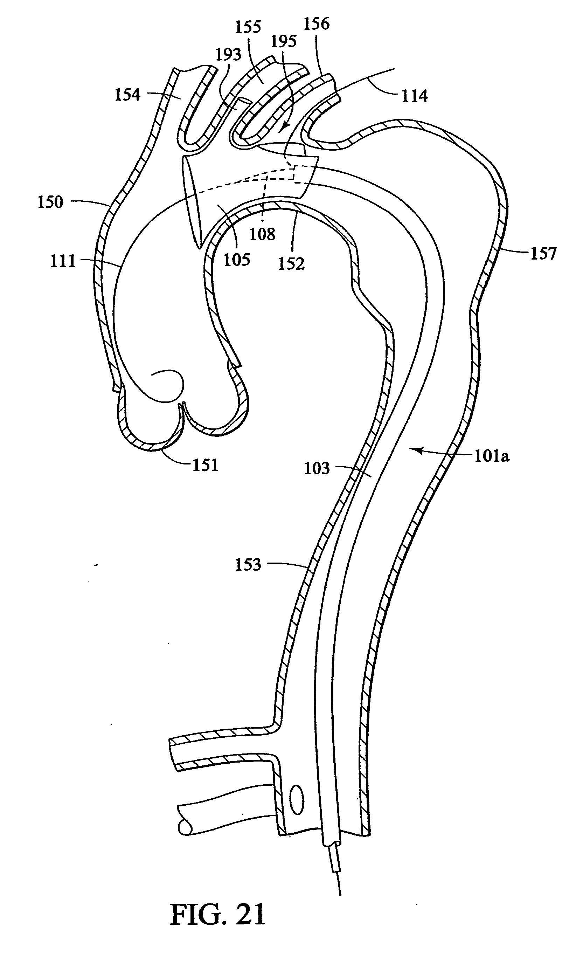

As can be seen in Figure 20 the introducer 101a has been introduced into the aorta normally via an incision into the femoral artery over a guide wire 111. The guide wire 111 has been deployed down towards the aortic valve 151. Once the introducer 101a is in position the auxiliary guide wire 114 is extended beyond the nose cone 108 of the introducer until it is adjacent the left subclavian artery. A suitable radio-graphic tip may be provided on the guide wire to assist with determination of its location. A prosthetic module 105 has previously been deployed in the aortic arch. A prosthetic branch 193 of the module 105 extends into the left carotid artery. The module 105 does not obstruct the left subclavian artery because of a notch 195 in the module 105, shown in Figure 21.

An incision can then be made into the brachial artery of the left arm and a snare catheter 160 introduced into the brachial artery and via that to the left subclavian artery and this snare catheter has a loop 161 at its end which can then be used to snare the guide wire 114. The snare is used to grip and pull the flexible guide wire 114 into the left subclavian artery and out through the brachial artery.

In the next stage shown in Figure 21 the introducer 101a is advanced proximally over the guide wire 111 with the auxiliary guide wire 114 still extending into the left subclavian artery. The introducer is advanced until the nose cone is adjacent the left subclavian artery and the sheath 103 is just distal of the opening of the artery into the aortic arch.

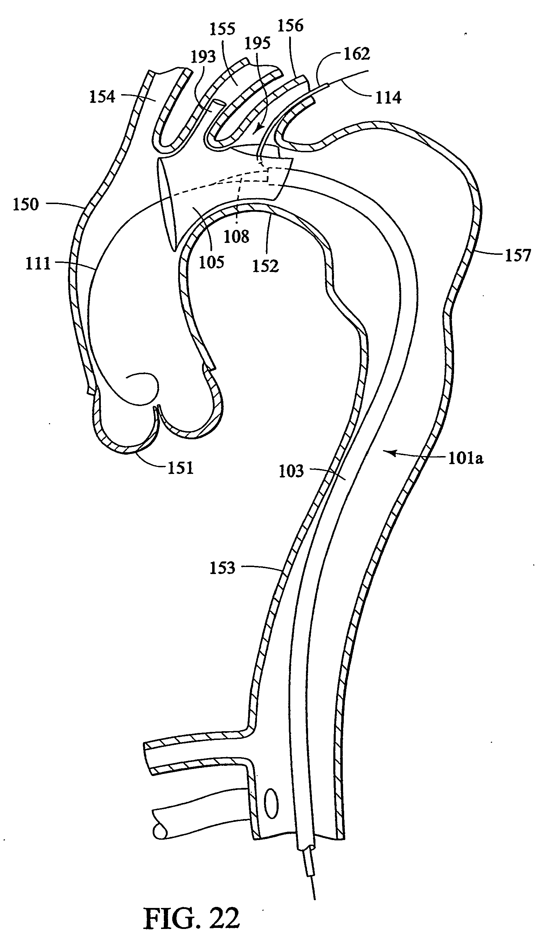

As can be seen in Figure 22 a protective catheter or sleeve 162 is deployed onto the auxiliary guide wire where it exits from the brachial artery and is then slid over the auxiliary guide wire 114 from the brachial artery end to protect the junction 157 of the left subclavian artery with the aortic arch during the subsequent steps. The protective catheter or sleeve 162 and the auxiliary guide wire 114 are then locked together so that they can be used to pull the prosthetic side branch 123 of the prosthetic module into the left subclavian artery 156.

As can be seen in Figure 23 the sheath 103 of the introducer 101a has been partially withdrawn so that the prosthetic module 120 retained by the sheath 103 has expanded but the retention arrangement 135 still holds the proximal end 121 of the graft in a restrained condition. The prosthetic side branch 123 is also released from the sheath but still held in a retracted condition by diameter reducing ties 129. It will be noted that at this stage the distal end 122 of the prosthetic module is still retained within the sheath 103.

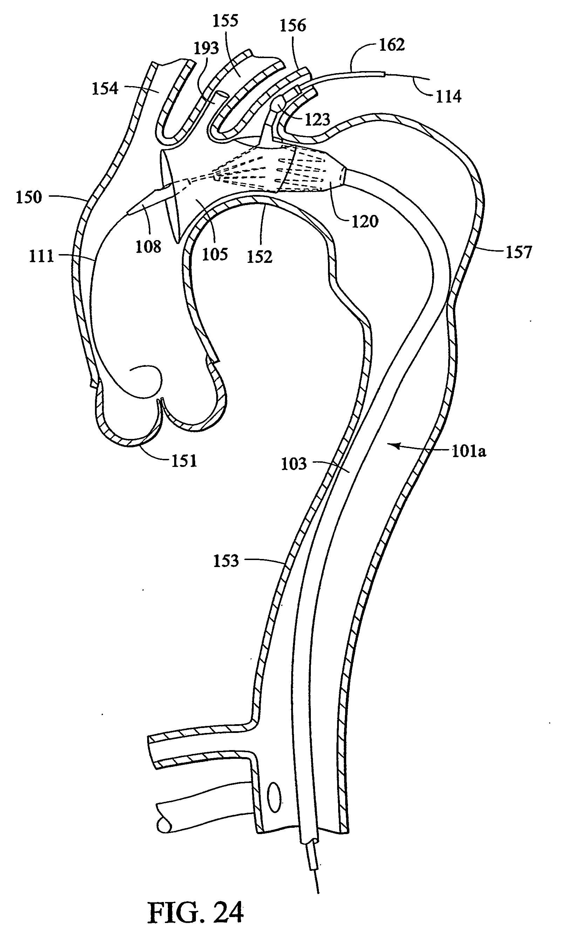

As shown in Figure 24, the introducer 101a is then moved further proximally while pulling on the protection catheter 162 and guide wire 114 so that the prosthetic side branch 123 of the prosthetic module 120 is pulled into the left subclavian artery 156.

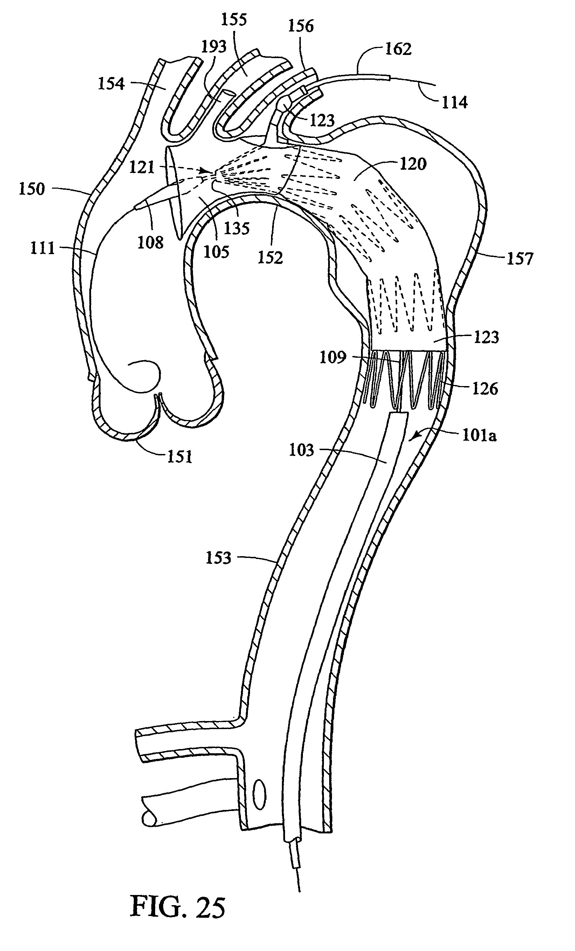

As shown in Figure 25 the sheath 103 is then pulled further back so that the distal end of the graft 120 is released from the sheath 103 and then the distal trigger wire release mechanism 140 (see Figure 11) can be released to release the external zigzag stents 126 so that they expand against the wall of the descending aorta 153.

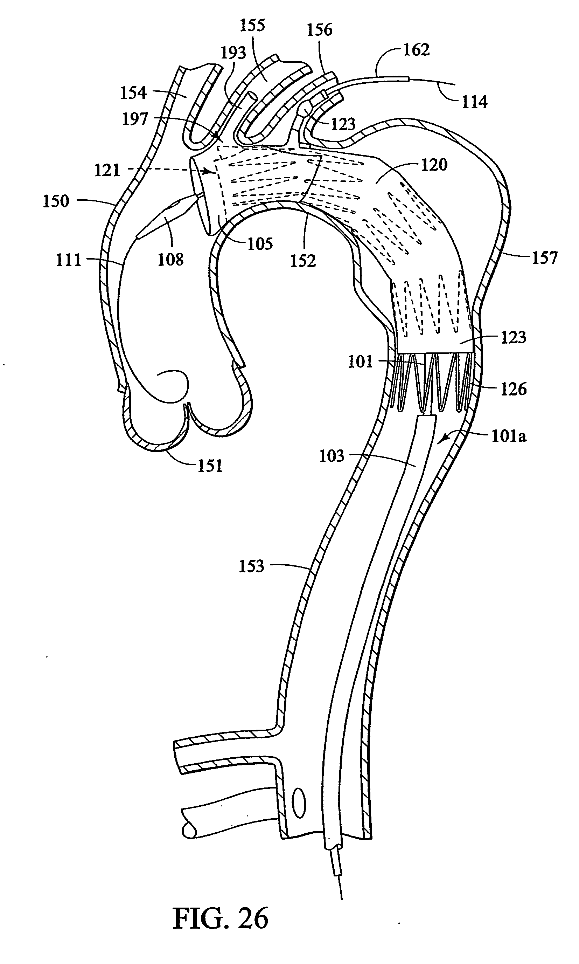

Next as shown in Figure 26 the proximal end 121 of the graft 120 is released by releasing the retention mechanism 135 (see Figure 11) by pulling on the trigger wire release mechanism 141 which pulls out trigger wire 136.

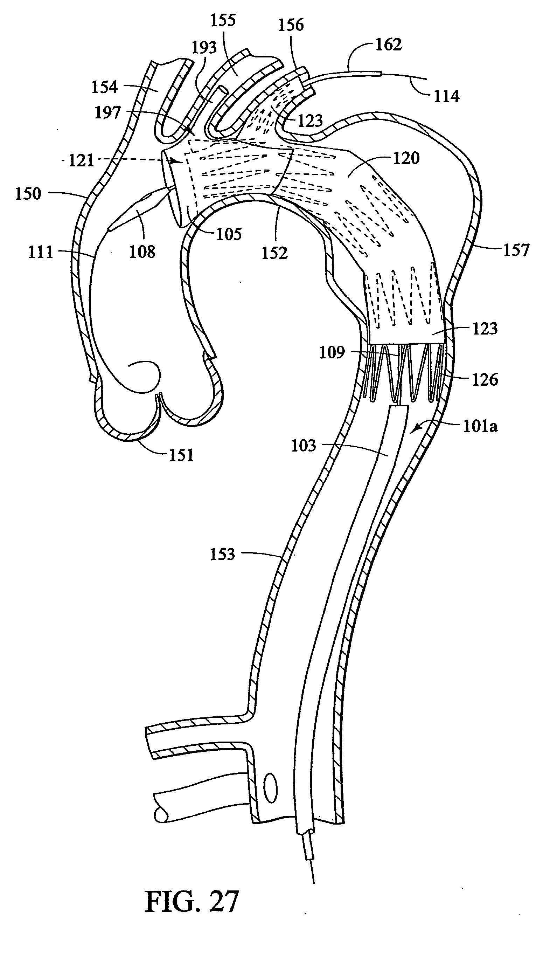

Finally as shown in Figure 27 the diameter reducing ties are released by pulling on the trigger wire release mechanism 142 (see Figure 11) which pulls the trigger wire 131.

The auxiliary guide wire 114 can then be retracted into the introducer 101a and the introducer removed from the aorta to leave the prosthetic module 120 deployed in the aorta with the prosthetic side branch 123 deployed into the left subclavian artery 156.

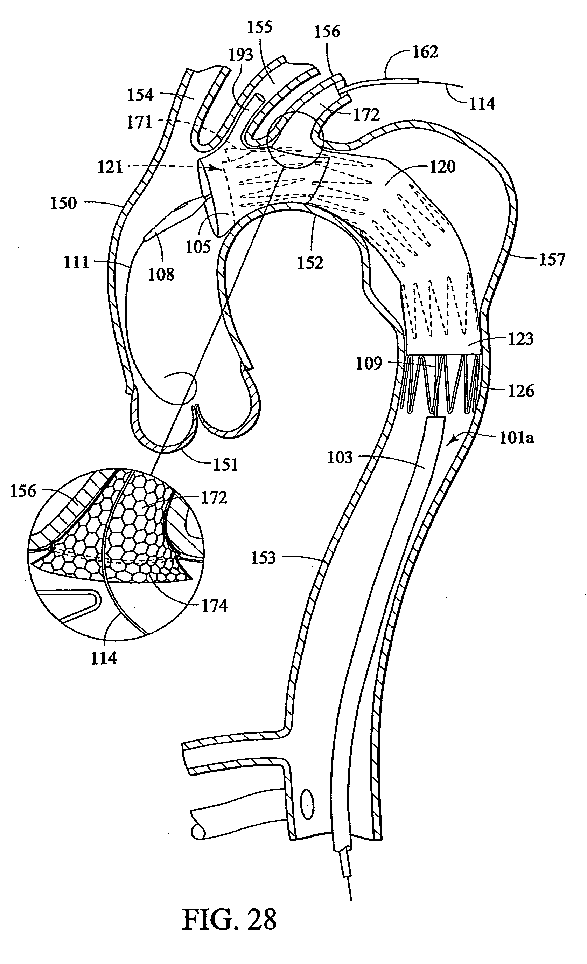

Figure 28 shows a schematic and a detailed view of a deployment stage of another embodiment of a prosthetic module into an aortic arch of a patient. After the introducer is deployed into the aortic arch and the auxiliary guide wire snared as shown in Figures 20 and 21 the introducer is further advanced proximally over the main guide wire. It will be noted that at this stage the notch 171 at the proximal end 121 of the prosthetic module 120 is positioned at the adjacent carotid artery 155 so as not to occlude it. This arrangement enables the proximal end 121 of the prosthetic module 120 to have a larger overlap with the telescoping module 105.

The prosthetic branch 172 can be deployed through the left subclavian artery over the auxiliary guide wire 114 and then it can be balloon expanded so that the ostium 174 expands to its full diameter.

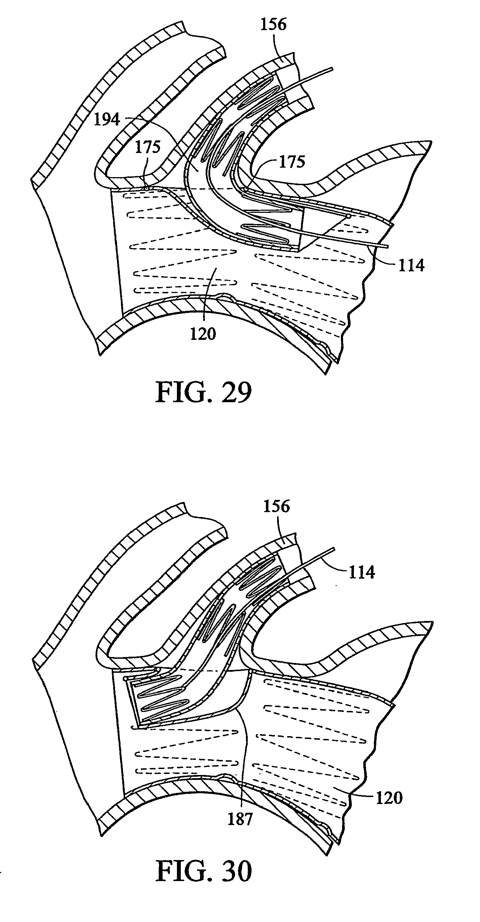

Figure 29 and 30 show detailed views of a deployment stage of another prosthetic module into an aortic arch of a patient. After the introducer is deployed into the aortic arch and the auxiliary guide wire snared as shown in Figures 20 and 21 the introducer is further advanced proximally over the main guide wire 111 (see Figure 21). A balloon expandable or self expanding prosthetic module 194 is then deployed into the left subclavian artery over the auxiliary guide wire 114 and then it can be allowed to expand or be balloon expanded.

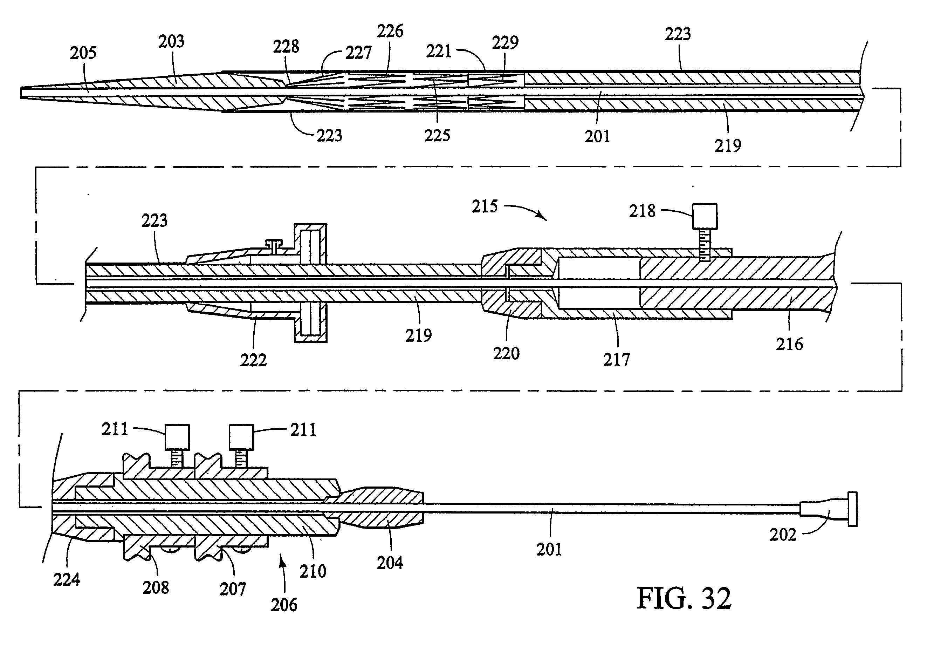

Now looking more closely at the drawings and in particular Figures 31 and 32, it will be seen that the introducer generally comprises, working from the inside towards the outside, a guide wire catheter 201 which extends the full length of the device from a syringe socket 202 at the far distal end of the introducer to a nose dilator 203 at the proximal end of the introducer.

The nose cone dilator 203 is fixed to the guide wire catheter 201 and moves with it. The nose cone dilator has a through bore 205 as an extension of the lumen of the guide wire catheter 201 so that the introducer can be deployed over a guide wire (not shown). To lock the guide wire catheter 201 with respect to the introducer in general, a pin vice 204 is provided.

The trigger wire release mechanism generally shown as 206 at the distal end of the introducer includes a distal end trigger wire release mechanism 207 and a proximal end trigger wire release mechanism 208. The trigger wire release mechanisms 207 and 208 slide on a portion of the fixed handle 210. Until such time as they are activated, the trigger wire mechanisms 207 and 208 which are fixed by thumbscrews 211 and remain fixed with respect to the fixed portion of the fixed handle.

Immediately proximal of the trigger wire release mechanism 206 is a sliding handle mechanism generally shown as 215. The sliding handle mechanism 215 generally includes a fixed handle extension 216 of the fixed handle 210 and a sliding portion 217. The sliding portion 217 slides over the fixed handle extension 216. A thumbscrew 218 fixes the sliding portion 217 with respect to the fixed portion 216. The fixed handle portion 216 is affixed to the trigger wire mechanism handle 210 by a screw threaded nut 224. The sliding portion of the handle 217 is fixed to the deployment catheter 219 by a mounting nut 220. A deployment catheter extends from the sliding handle 217 through to a capsule 221 at the proximal end of the deployment catheter 219.

Over the deployment catheter 219 is a sheath manipulator 222 and a sheath 223, which slides with respect to the deployment catheter 219 and in the ready to deploy situation as shown in Figures 31 and 32 extends from the sheath manipulator 222 forward to the nose cone dilator 203 to cover a prosthetic module 225 retained on the introducer distally of the nose cone dilator 203.

In the ready to deploy condition shown in Figures 31 and 32, the sheath 223 assists in retaining the prosthetic module 225, which includes self-expanding stents 226 in a compressed condition. The proximal covered stent 227 is retained by a fastening at 228 which is locked by a trigger wire (not shown) which extends to trigger wire release mechanism 208. The distal exposed stent 229 on the prosthetic module 225 is retained within the capsule 221 on the deployment catheter 219 and is prevented from being released from the capsule by a distal trigger wire (not shown) which extends to the distal trigger wire release mechanism 207.

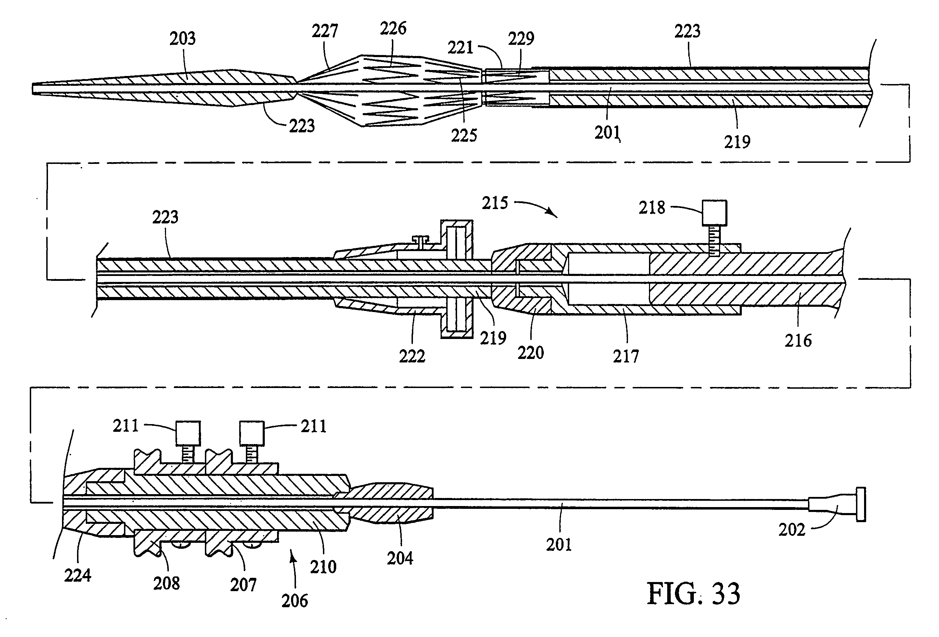

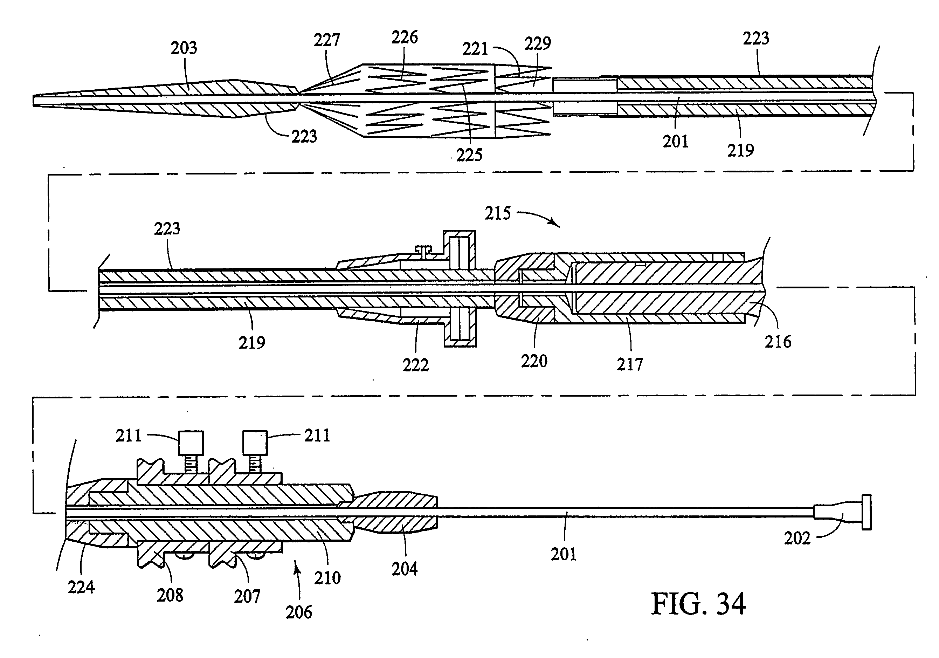

Figure 33 shows the same view as Figure 32 but after withdrawal of sheath 223, and Figure 34 shows the same view as Figure 33, but after activation of sliding handle mechanism 215.

In Figure 33, the sheath manipulator 222 has been moved distally so that its proximal end clears the prosthetic module 225 and lies over the capsule 221. Freed of constraint, the self expanding stents 226 of the prosthetic module 225 are able to expand. However, the fastening 228 still retains the proximal end of the proximal stent 227, and the capsule 221 still retains the distally extending exposed stent 229. At this stage, the proximal and distal ends of the prosthetic module 225 can be independently repositioned, although if the proximal stent 227 included barbs as it has in some embodiments, the proximal end can only be moved proximally.

Once repositioning has been done, the distal end of the prosthetic module 225 should be released first. This is done so that blood flow, which is from proximal to distal, cannot inflate the prosthetic module in a wind sock type of effect and cause migration of the prosthetic module during deployment. For this reason, it is desirable to release the distal end of the prosthetic module first, but if the capsule is moved distally, then the release mechanisms could also move, which could release the proximal end prematurely. Hence the distal trigger wire release mechanism 207 on the handle 210 is removed to withdraw the distal trigger wire. Then the thumb screw 218 is removed, and the sliding handle 217 is moved distally to the position shown in Figure 34. This moves the capsule 221 to release the exposed stent 229. As the fastening 228 is retained on the guide wire catheter 201, just distal of the nose cone dilator 203 and the guide wire catheter 201 is locked in position on the handle 210 by pin vice 204, then the proximal trigger wire release mechanism 208, which is on the handle 210, does not move when moving the sliding handle, deployment catheter 219 and capsule 221 so the proximal end of the prosthetic module 225 remains in a retained position. The proximal end of the prosthetic module 225 can be again manipulated at this stage by manipulation of the handle. Although if the proximal stent 227 included barbs as discussed above, the proximal end can only be moved proximally. The proximal fastening 228 can then be released by removal of the proximal trigger wire release mechanism 208.

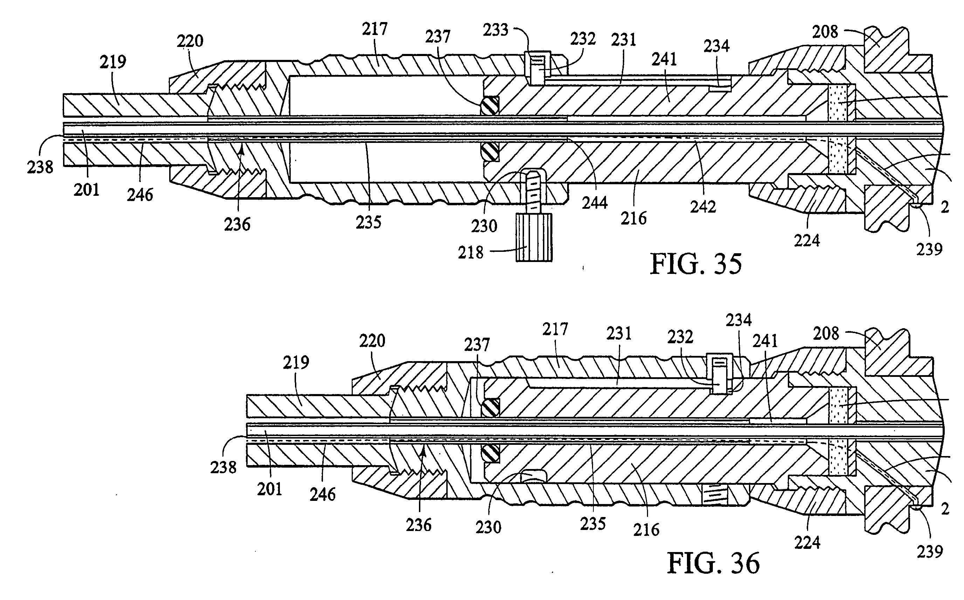

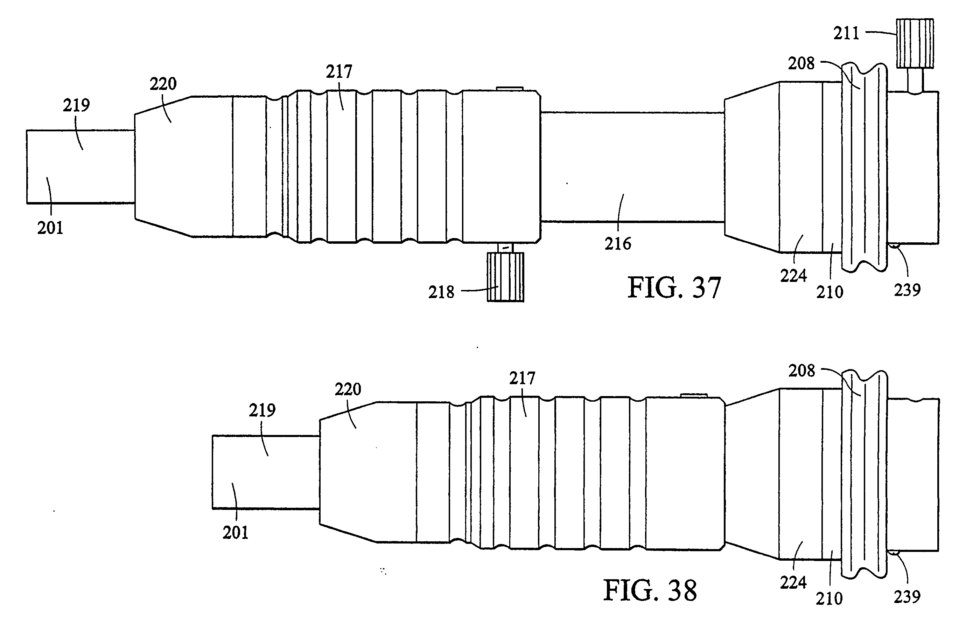

Now looking more closely at Figures 35 to 38, the detailed construction of a particular embodiment of a sliding handle mechanism according to this invention is shown. Figures 35 and 37 show the sliding handle mechanism in the ready to deploy condition. Figures 36 and 38 show the mechanism when the deployment catheter and hence the capsule has been withdrawn by moving the sliding handle with respect to the fixed handle. The fixed handle extension 216 is joined to the trigger wire mechanism handle 210 by screw threaded nut 224.

The sliding handle 217 is fixed to the deployment catheter 219 by screw threaded fixing nut 220 so that the deployment catheter moves along with the sliding handle 217. The sliding handle 217 fits over the fixed handle extension 216 and, in the ready to deploy situation, is fixed in relation to the fixed handle by locking thumbscrew 218, which engages into a recess 230 in the fixed handle extension 216. On the opposite side of the fixed handle extension 216 is a longitudinal track 231 into which a plunger pin 232 spring loaded by means of spring 233 is engaged. At the distal end of the track 231 is a recess 234.

A guide tube 235 is fixed into the proximal end of the sliding handle 217 at 236 and extends back to engage into a central lumen 241 in the fixed handle extension 216 but able to move in the central lumen 241. An O ring 237 seals between the fixed handle extension 216 and guide tube 235. This provides a hemostatic seal for the sliding handle mechanism. The trigger wire 238, which is fixed to the trigger wire releasing mechanism 208 by means of screw 239, passes through the annular recess 242 between the fixed handle extension 216 and the guide wire catheter 201 and then more proximally in the annular recess 244 between the guide wire catheter 201 and the guide tube 235 and forward to extend through the annular recess 246 between the guide wire catheter 201 and the deployment catheter 219 and continues forward to the proximal retaining arrangement. Similarly the distal trigger wire (not shown) extends to the distal retaining arrangement.

A further hemostatic seal 240 is provided where the guide wire catheter 201 enters the trigger wire mechanism handle 210 and the trigger wires 238 pass through the hemostatic seal 240 to ensure a good blood seal.

As can be seen in Figures 36 and 38, the locking thumbscrew 218 has been removed and discarded, and as the sliding handle is moved onto the fixed handle, the plunger pin 232 has slid back along the track 231 to engage into the recess 234. At this stage, the sliding handle cannot be moved forward again.

As the trigger wire release mechanisms 207 and 208 are on the trigger wire mechanism handle 210, which is fixed with respect to the fixed handle 216, then the proximal trigger wire 238 is not moved when the deployment catheter 219 and the sliding handle 217 is moved so that it remains in position and does not prematurely disengage.

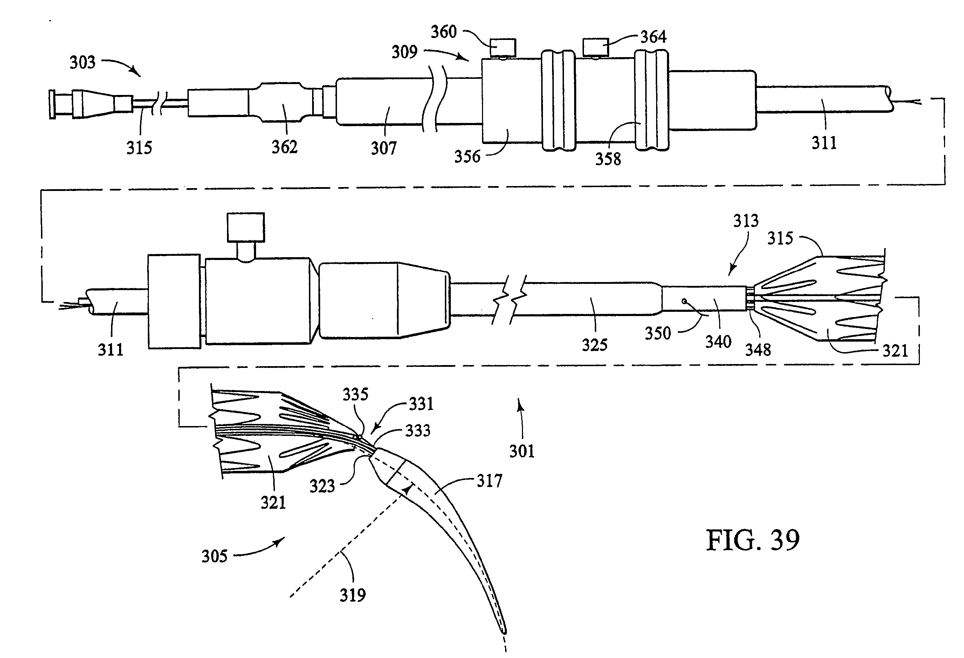

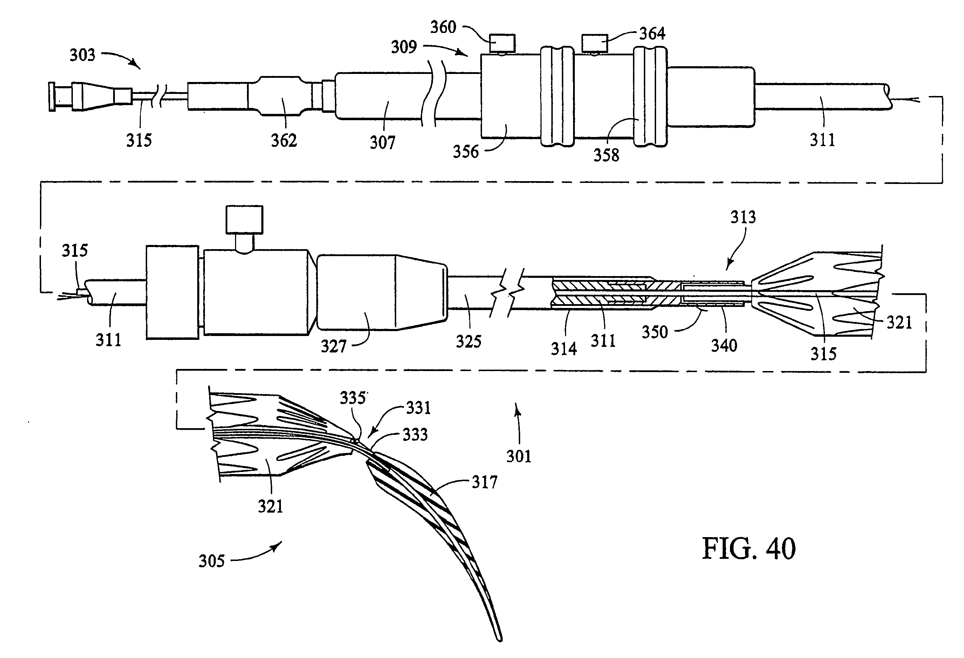

Now looking more closely at Figures 39 and 40 it will be seen that a prosthetic module introducer 301 according to the invention has a distal end 303 which in use is intended to remain outside a patient and a proximal end 305 which is introduced into the patient. This introducer is further described in U.S. Patent Application Serial No. 10/609,842, filed June 30, 2003.

Towards the distal end there is a handle arrangement 307 which includes trigger wire release apparatus 309 as will be discussed later. The main body of the introducer includes a tubular carrier 311 which extends from the handle 307 to a distal retention arrangement general shown as 313.

Within a longitudinal lumen 314 in the central carrier 311 extends a guide wire catheter 315. The guide wire catheter 315 extends out through the distal retention arrangement 313 and extends to a nose cone dilator 317 at the proximal end of the introducer 301. The nose cone dilator 317 is curved and in the embodiment shown in Figures 39 the guide wire catheter 315 is also curved towards its proximal end so that the proximal end 305 of the introducer has a curve which may have a radius of curvature 319 of between 70 to 150 millimeters. This curvature enables the prosthetic module introducer of the present invention to be introduced into the aortic arch of a patient without excessive load being placed on the walls of the aorta.

A prosthetic module 321 is retained on the introducer between the distal end 323 of the nose cone dilator 317 and the distal retention arrangement 313. A sleeve 325 fits over the tubular carrier 311 and by operation of a sleeve manipulator 327 the sleeve can be extended forward to extend to the nose cone dilator 317. By the use of the sleeve 325 the prosthetic module 321 can be held in a constrained position within the sleeve.

At the proximal end of the prosthetic module just distal of the distal end 323 of the nose cone dilator 317 a proximal retention arrangement 331 is provided.

The proximal retention arrangement 331 includes a trigger wire 333 which engages a knot 335 of suture material which is fastened to the trigger wire 333 and the guide wire catheter 315. When the trigger wire 333 is withdrawn as will be discussed later, the suture knot 325 is released and the proximal end of the prosthetic module can be released. The nose cone dilator 317 can have one or more apertures extending longitudinally, and the proximal trigger wire 333 can extend into one of these apertures.

The distal retention arrangement 313 as shown in detail in Figures 40 includes a capsule 340 which is part of a capsule assembly 341 which is joined by a screw thread 343 to the proximal end 342 of the central carrier 311. The capsule 340 includes a passageway 344 within it with a distal closed end 346 and an open proximal end 348. The open proximal end 348 faces the nose cone dilator 317 and the guide wire catheter 315 passes through the center of passageway 344.

The prosthetic module 321 has a distally extending exposed stent 348 and this distally extending exposed stent 348 is received within the capsule 340 which holds it constrained during deployment. If the distally extending exposed stent 348 has barbs extending from its struts then the capsule keeps the barbs from prematurely engaging the walls of the vessel it is being deployed in and also prevents them from catching in the sleeve 325. A trigger wire 350 passes through aperture 352 in the side of the capsule, engages a loop of the exposed stent 348 within the capsule and then passes along the annular recess 354 between the guide wire catheter 315 and the tubular carrier 311 to the trigger wire release mechanism 309.

The trigger wire release mechanism 309 includes a distal release mechanism 356 and a proximal end release mechanism 358.

To release the prosthetic module after it has been placed in the desired position in the aortic arch, the sleeve 325 is withdrawn by pulling back on the sleeve manipulator 327 while holding the handle 307 stationary. The distal release mechanism 356 on the handle 307 is then released by loosening the thumb screw 360 and completely withdrawing the distal release mechanism 356 which pulls out the trigger wire 350 from the capsule 340. Pin vice 362 which fixes the position of the guide wire catheter with aspect to the handle 307 and central carrier 311 is then loosened so that the guide wire catheter 315 can be held stationary which holds the nose cone dilator and hence the proximal retention arrangement 331 stationary while the handle is pulled back to remove the capsule 340 from the exposed stent 348 which releases the distal end of the prosthetic module.

Once the position of the proximal end of the prosthetic module 321 has been checked, the proximal release mechanism 358 can then be removed by release of the thumb screw 364 and complete removal of the proximal release mechanism 358 which pulls the guide wire 333 from the proximal end of the prosthetic module which releases the suture knot 335 which releases the proximal end of the prosthetic module.

The tubular central carrier 311 can then be advanced while holding the nose cone dilator 317 stationary so that the introducer can be made more compact for withdrawal.

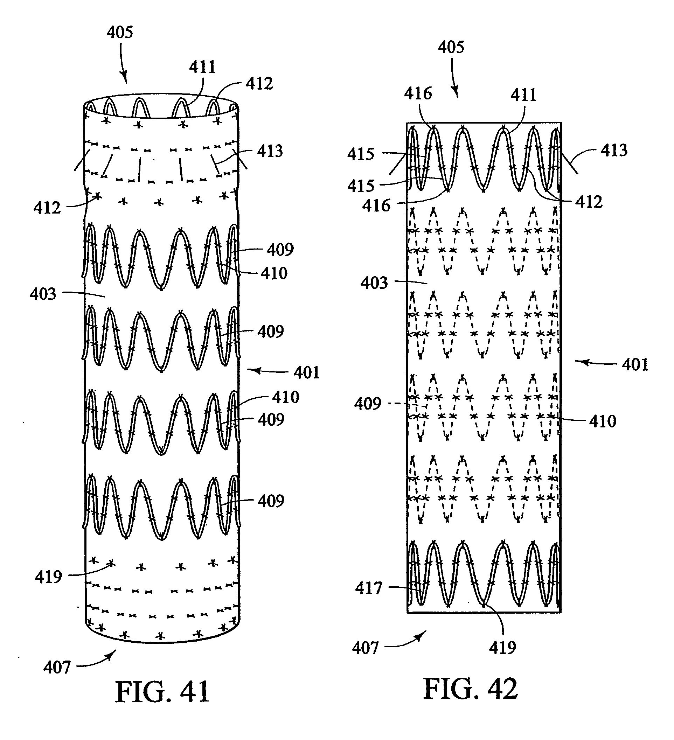

Now looking more closely at the drawings and in particular Figures 41 and 42 showing external and internal views of a first embodiment of a prosthetic module similar to that described in relation to Figure 1f, it will be seen that a prosthetic module 401 includes a tubular body 403 formed from a biocompatible woven or non-woven fabric or other material. The tubular body has a proximal end 405 and a distal end 407. The tubular body may have a diameter in the range of 10 mm to 60 mm and a length of from 50 mm to 500 mm. The prosthetic module 401 may be tapered, outwardly bulging like a balloon or of constant diameter along its length depending upon the topography of the vasculature.

Along the length of the tubular body, there are a number of self-expanding zigzag stents 409 such as the well-known Gianturco Z or zigzag stent on the outside of the body. In this embodiment there are four external stents 409 spaced apart by a distance of between 5 mm to 10 mm. The external stents 409 are joined to the graft material by means of stitching 410 preferably using a monofilament or braided suture material.

At the proximal end 405 of the prosthetic module 401 there is provided an internal zigzag stent 411 which provides a sealing function for the proximal end of the prosthetic module. The outer surface of the tubular body 403 at the proximal end 405 presents an essentially smooth outer surface which with the assistance of the internal zigzag stent 411 can engage and seal against the wall of the aorta when it expands and is deployed. The proximal stent 411 is comprised of struts 415 with bends 416 at each end of the struts. Affixed to some of the struts 415 are barbs 413 which extend distally from the struts through the graft material. When the prosthetic module is deployed into an aortic arch, the barbs 413 engage and/or penetrate into the wall of the aorta and prevent distal movement of the prosthetic module caused by pulsating blood flow through the prosthetic module.

It will be noted that the stent 411 is joined to the graft material by means of stitching 412 preferably using a monofilament or braided suture material.

At the distal end 407 of the prosthetic module 401, there is an internal sealing stent 417 (see Figure 42) which again is fastened to the graft material body 403 by stitching 419 preferably using a monofilament or braided suture material. The outer surface of the tubular body 403 at the distal end 407 presents an essentially smooth outer surface which with the assistance of the internal zigzag stent 417 can engage and seal against the wall of the aorta when it is deployed.

The prosthetic module shown in Figures 41 and 42 may be used for treatment of patients with symptomatic acute or chronic dissections and ruptures in the descending thoracic aorta and may be connected to one or more of the branched prosthetic modules described above.

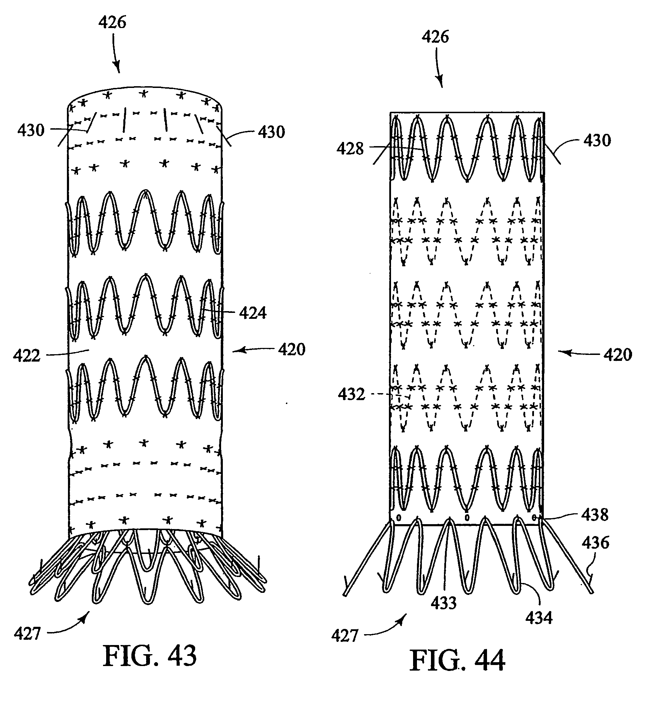

Figures 43 and 44 show external and internal views of a second embodiment of the prosthetic module according to the present invention. In this embodiment, the prosthetic module 420 has a tubular graft material body 422 in the same manner as the embodiment shown in Figures 41 with external stents 424 spaced along the body with a longitudinal spacing of approximately 5 mm to 10 mm between the stents. The length of the prosthetic module may be in the range of 50 mm to 500 mm and a diameter in the range of 10 mm to 60 mm in 2 mm increments.

Also in a similar manner to the embodiment shown in Figures 41 and 42 at the proximal end 426 of the prosthetic module there is an internal sealing stent 428 with barbs 430.

At the distal end 427 of the prosthetic module 420, there is also an internal distal sealing stent 432 but in addition, there is a distally extending exposed zigzag stent 434. This distally extending exposed stent 434 has barbs 436 on some of its struts and these barbs 436 are directed proximally. The distally extending exposed zigzag stent 434 is fastened to the tubular graft material body 422 by stitching 433.

It will be noted that there are provided at the distal end of the prosthetic module radiographic markers 438 to enable correct positioning of the distal end of the prosthetic module.

Hence, when the prosthetic module according to this embodiment of the invention is deployed, the barbs 430 prevent distal migration of the proximal end of the prosthetic module and the barbs 436 on the exposed stent 434 prevent proximal migration of the distal end of the prosthetic module 420. This tendency of distal migration of the distal end and proximal migration of the distal end may occur if the central portion of the prosthetic module is free within an aneurysm and sideways force on a curved prosthetic module caused by pulsating blood flow causes sideways movement of the body of the prosthetic module with the potential for distal movement of the proximal end and proximal movement of the distal end.

The prosthetic module shown in Figures 43 and 44 may be used for endovascular repair of thoracic aortic aneurysms in the descending thoracic aorta. Additional prosthetic modules are described in U.S. Patent Application Serial No. 10/396,676, filed March 25, 2003 and U.S. Patent Application Serial No. 10/609,835, filed June 30, 2003.

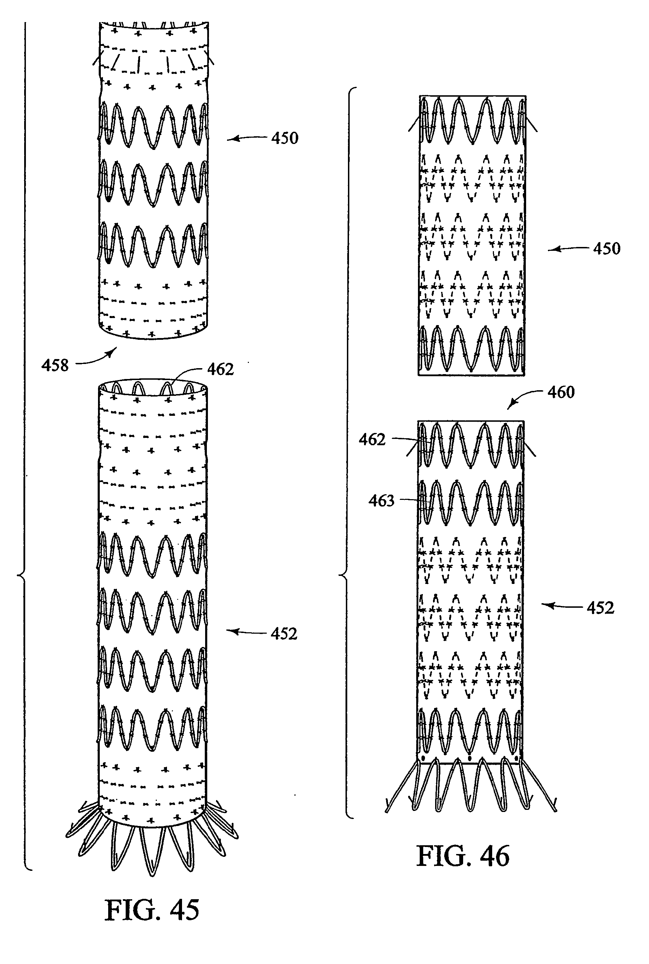

Figures 45 and 46 show external and internal views of a third embodiment of the prosthetic module according to the present invention. In this embodiment, a thoracic composite prosthesis is formed from a first portion 450 and a second portion 452. The first portion 450 is intended to be deployed proximally of the second portion 452. The first portion 450 is substantially identical with the prosthetic module embodiment shown in Figures 41 and 42. It has proximal and distal internal sealing stents in a tubular graft body, barbs extending from the proximal sealing stent and external zigzag stents between the proximal and distal sealing stents.

The second portion 452 is substantially the same as the embodiment shown in Figures 43 and 44 except that there are two internal sealing stents 462, 463 at the proximal end 460 of the second portion 452. It will be noted that although the second portion 452 is substantially similar to the embodiment shown in Figures 43 and 44 it does not include the distally extending anchoring barbs on the proximal sealing stent 462.

The proximal end 460 of the second portion 452 with the internal sealing stents 462, 463 can be deployed either inside the distal end 458 of the first portion 450 or outside the distal end 458 of the first portion 450. This means that in deploying the composite prosthesis of this embodiment of the invention either the first or second portions may be deployed first and the other portion subsequently deployed depending upon the requirements in a particular case.

In either case it is preferable to have at least two stents overlap and it may be noted that by having this overlap there is at least one stent length of smooth internal surface of one of the portions engaging against a smooth external surface of the other of the portions. By this arrangement, sealing between the first and second portions is possible. Also, by having an overlap of at least two stents, relative movement between the first and second portions is less likely to cause parting of the first and second portions of the thoracic composite prosthesis when it is deployed and pulsating blood flow through the prosthetic module causes sideways movement of the centre portion of the prosthetic module as discussed above.

The stent graft shown in Figures 45 and 46 may be used for endovascular repair of thoracic aortic aneurysms in the descending thoracic aorta and particularly for treatment of patients with atherosclerotic aneurysms, symptomatic acute or chronic dissections, contained ruptures and growing aneurysms. The ability to adjust the overall length of the device by providing more or less overlap of the first and second portions (i.e. "telescoping") allows more accurate placement of the proximal and distal sealing stents and the anchoring barbs.

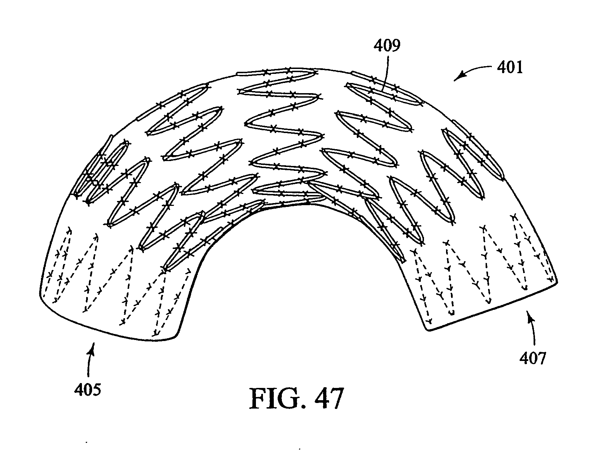

Figures 47 shows a prosthetic module of the embodiment shown in Figures 41 to show the amount of bending which is possible in the prosthetic module for placement in the aortic arch of a patient. The internal radius of curvature of the prosthetic module according to this invention may be any radius greater than 35 mm. This can be achieved by having the stents longitudinally spaced apart by between 5.0 mm to 10.0 mm and so far as possible staggering the placement of apices of adjacent stents. This may not be possible where adjacent stents have different numbers of struts.

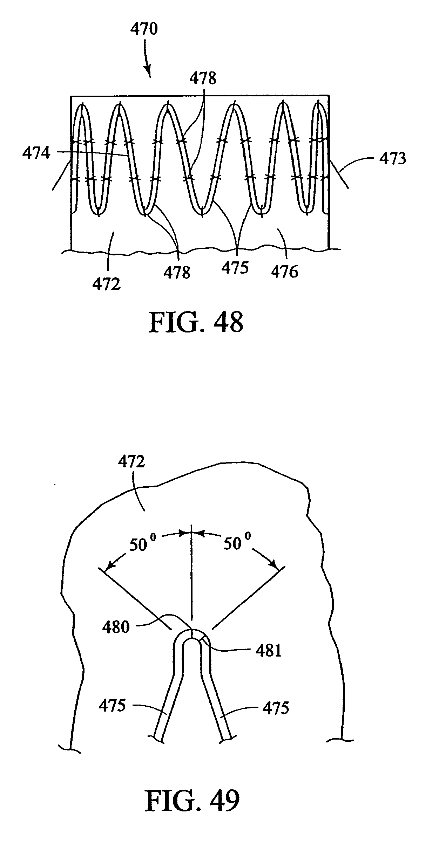

Figure 48 shows features of a sealing stent that may be present in any of the above embodiments. A prosthetic module 470 has a graft material body 472 and an internal sealing stent 474 joined to the graft material by stitching 478, also referred to as fastenings. Stent 474 is a zigzag stent having struts 475 with bends 476 at each end of the struts. Affixed to at least some of the struts 475 are barbs 473 which extend distally from the struts through the graft material.

As described in U.S. Provisional Patent Application Serial No. 60/391,737, where the prosthetic module is deployed in a blood vessel, blood flow causes a pull on the graft material tube which is resisted by the barbs on the stent. Hence the fastenings of the stent joining the stent to the graft material take the pull on the prosthesis, and these fastenings preferably are sufficiently strong to take that pull. Similarly, the barbs on the exposed stent used on the distal end of the graft material tube resist blood flow pull on the graft material tube.

Figure 49 shows features of a distally extending exposed zigzag stent used in the embodiments shown in Figures 43 to 46. As can be seen in the detailed views in Figures 49, the struts 479 and bend 482 of the stent are on the inside of the graft material 472. At least two fastenings 480 and 481 are used to fasten the stent 483 to the graft material 472. As shown here, the first fastening 480 is positioned at the apex of the bend 482, and a second fastening 481 is positioned spaced apart adjacent the transition from the bend 482 to the struts 479 of the stent. The second fastening 481 can be positioned on either strut 479 extending from the bend 482 in a region extending up to an angle of 50° either side of the first fastening 480 measured around the radius of the bend from the apex of the bend 482. Generally the second fastening 481 is spaced from the first fastening 480 by 0.5 mm to 2 mm. The spaced apart fastenings are preferably present at the proximal bends of the distally extending stent 434 of Figures 43 and 44, and the distally extending stent of Figures 45 and 46.

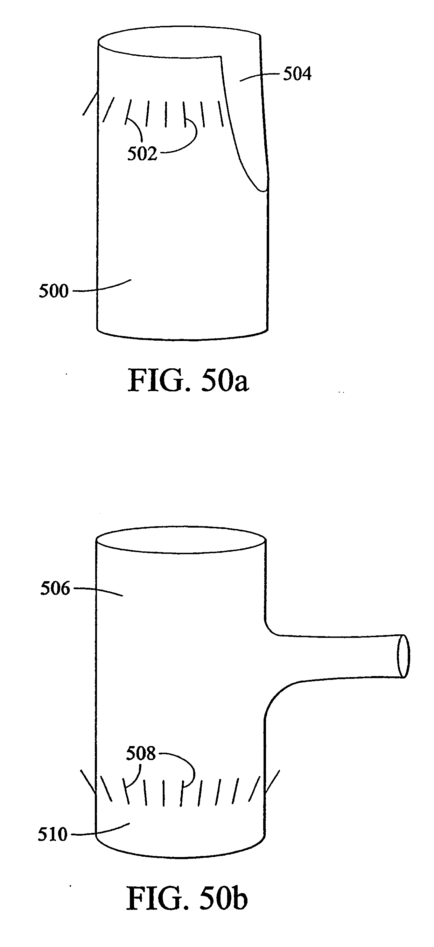

Additional means may be used to increase the pull-out force between the prosthetic modules. For example, the inner module at a telescoping interconnection can have outward facing barbs (or hooks), as shown in Figures 50a and 50b. The module 500 in Figure 50a is designed to be an inner module that has outward and distally pointed barbs 502. The barbs 502 may engage an outer, previously deployed module to which it interconnects, and they may even pierce the outer module to engage the vessel wall to prevent migration of the entire prosthesis. These barbs may engage the fabric or graft material of the outer module and/or the stent frame of the outer module. The notch 504 may be aligned with a branch vessel as described above. Similarly, barbs may point proximally and out, as the barbs 508 do on the distal end 510 of a proximal module 506 shown in Figure 50b, when a primary purpose is preventing proximal migration or separation of the that module 506 from a distally connected one. Barbs may also be designed to anchor the modules relative to the vessel, like the barbs 427 shown in Figure 43.

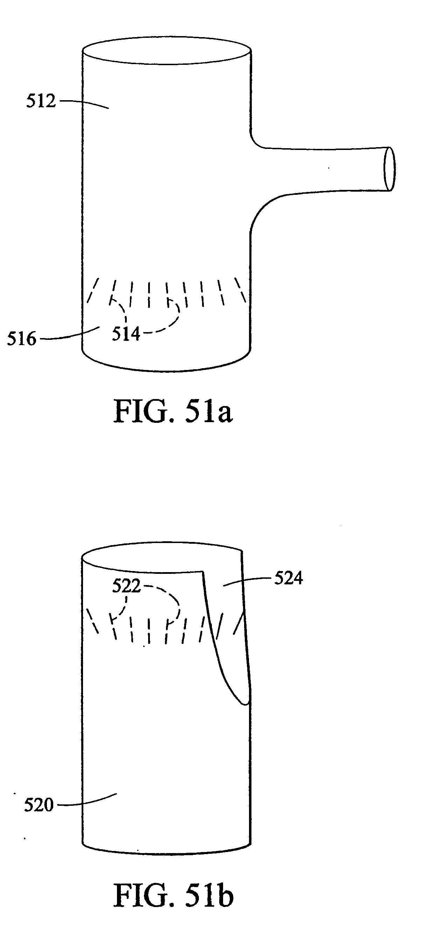

Barbs may also point inwardly on a module as shown in Figures 51a and 51b. The barbs 514, 522 on these modules 512, 520 may help prevent relative migration on an internally deployed interconnecting module. The barbs 514, 522 may be placed on the distal 516 end of the module 512 or on the proximal end 524 of the module 520 and can point distally or proximally depending on the forces to be opposed.

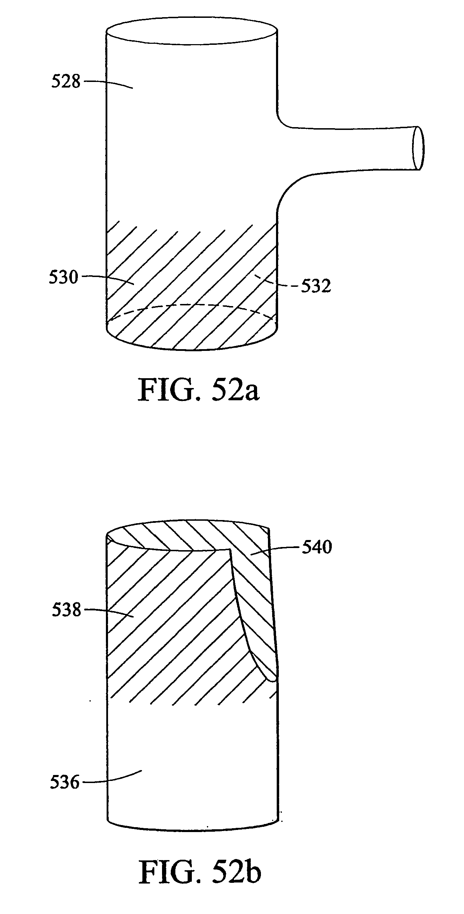

Other methods of improving fixation can be employed. For example, a one- or two-part glue or epoxy may be used to enhance fixation of two modules to each other or even a module to the surrounding vessel wall. Likewise, a two-part hook-and-loop connection (such as Velcro^{™}) may be employed to enhance fixation. As shown in Figures 52a and 52b, these materials can be added to the external surface 530 or an internal surface 532 of a prosthetic module 528, so that they contact an internally or externally placed interconnecting module. For example, the outer surface 530 of one module 528 could be coated with one part of a two-part glue (comprising a base and catalyst); this module could be connected to another module having the other part of the two-part glue on its internal surface 540 so that the two modules 528, 536 become adhered to one another when the end 538 of the one module 536 overlaps the other module 528. The two-part glue is preferably biodegradable as separate components, yet relatively permanent when a bond is formed. The glue could also be a sticky or tacky substance that adheres one module to another.

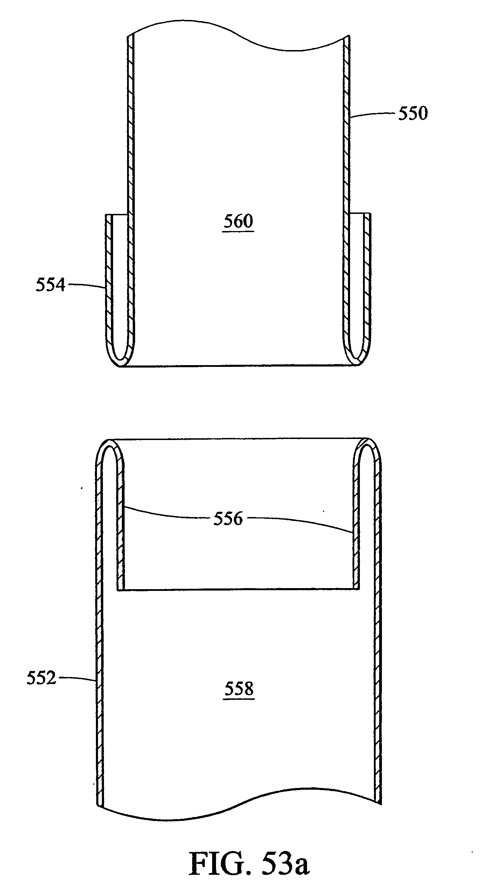

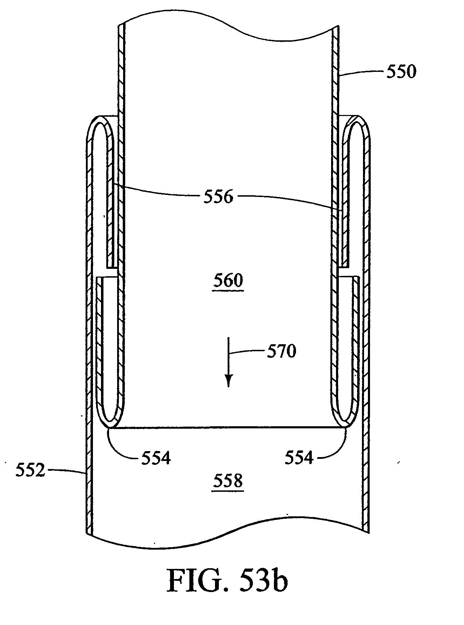

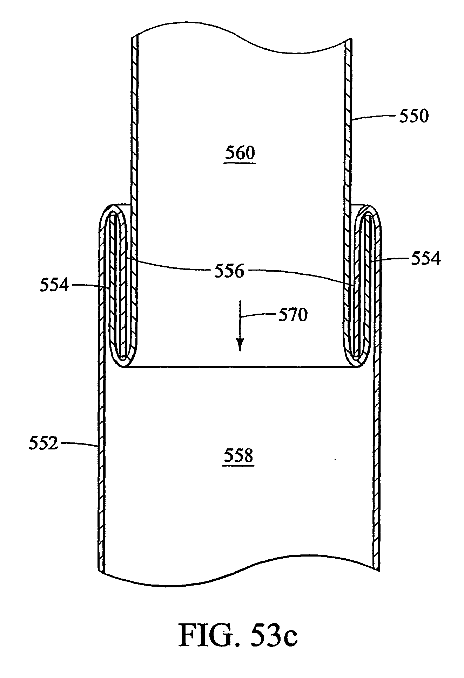

Mutually interlocking cuffs may also be used to strengthen the connection between modules. A cross-section of two modules 558, 560 is shown in Figure 53a. One module 560 has an external cuff 554 while the other module 558 has an internal cuff 556. Figure 53b shows the position of the cuffs 556, relative to another when one module 560 is deployed within the other module 558. The frictional forces of the blood flow 570 encourage the migration of the two modules 558, 560 away from one another so that the cuffs 554, 556 mutually engage, as shown in Figure 53c. The pressure within the module 558, 560 will compress the interlocking cuffs 554, 556, further enhancing the interconnection.

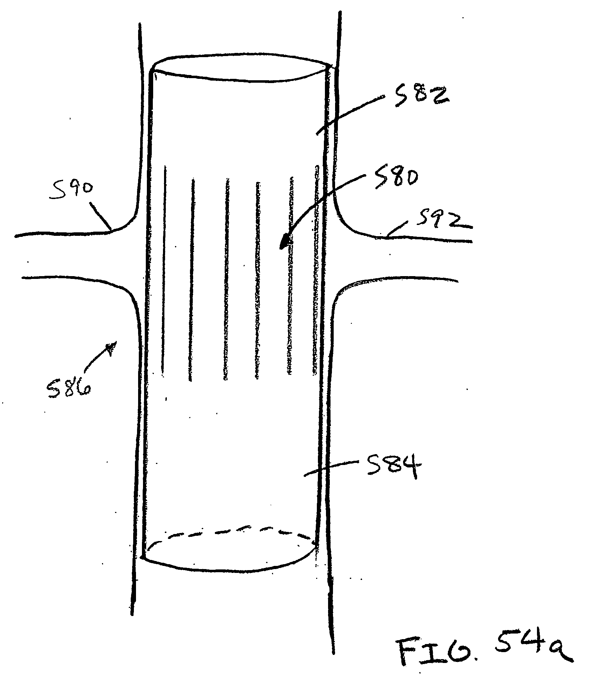

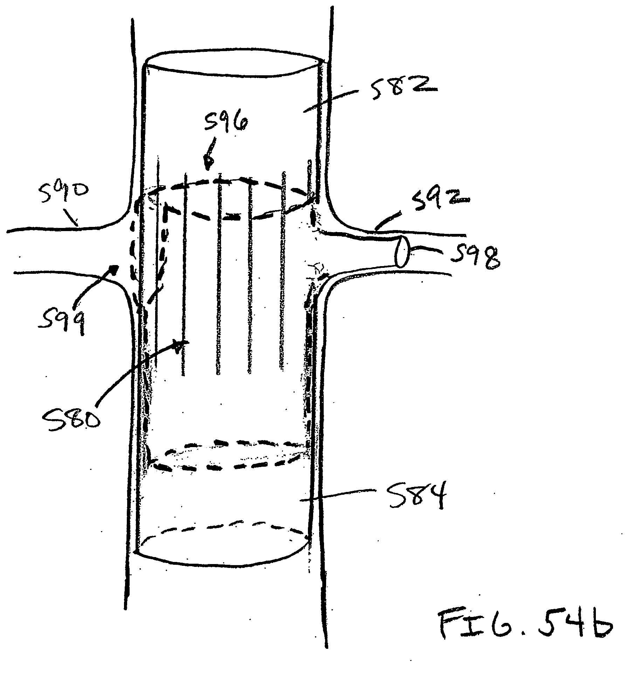

One or more of the prosthetic modules described above may be deployed within an existing module or prosthesis such that an integral prosthetic branch extends through a zone in the preexisting module that is composed only of warp fibers. For example, as shown in Figure 54a, there is a zone 580, shown schematically, that consists only of longitudinal warp fibers extending between two sections 582, 584 of the module 586 that consist of ordinary woven fabric. The warp zone 580 transverses the renal branches 590, 592. The warp zone 580, because it is not constrained by weft fibers or the like, does not pose a significant barrier to the deployment of a branch that extends through it. Thus, as shown in Figure 54b, a module 596 having an integral branch 598 and a notch 599 is deployed such that the integral branch 598 penetrates the warp zone 580 and enters a renal branch 592. An additional renal module may be deployed so that a second integral branch enters the other renal branch 590, as described above.

It is therefore intended that the foregoing detailed description be regarded as illustrative rather than limiting, and that it be understood that it is the following claims, including all equivalents, that are intended to define the scope of this invention.

## Claims

1. A modular endoluminal prosthesis for deployment in a body lumen, comprising:
a first prosthetic module (2) and a second prosthetic module (2);
the first prosthetic module comprising a first prosthetic trunk (3) for location within a main vessel, integral with a first prosthetic side branch (4) for location in a side branch vessel; and
the second prosthetic module comprising a second prosthetic trunk for location within a main vessel, the second prosthetic trunk having a first notch (11), the first notch arranged such that when the first and second prosthetic modules are interconnected, the first notch and the first prosthetic side branch are aligned;
wherein the first prosthetic side branch is for location in a side branch vessel that branches off a side of a main vessel; and
wherein a telescoping interconnection between the first prosthetic trunk and the second prosthetic trunk is established when the first and the second prosthetic modules are interconnected, such that the device may be adjusted by providing more or less overlap between the first prosthetic trunk and the second prosthetic trunk.

2. A prosthesis as claimed in claim 1, wherein the first notch is one of a fenestration and a scallop.

3. A prosthesis as claimed in claim 1 or 2, wherein the first notch (11) is provided at an end of the second prosthetic trunk.

4. A prosthesis as claimed in claim 1, 2 or 3, further comprising a second prosthetic side branch (42) integral with the second prosthetic module.

5. A prosthesis as claimed in claim 4, wherein the first prosthetic side branch and the second prosthetic side branch are generally on opposite sides of the prosthesis.

6. A prosthesis as claimed in claim 4, wherein the first prosthetic side branch and the second prosthetic side branch are generally on a single side of the prosthesis.

7. A prosthesis as claimed in claim 4, 5 or 6, wherein the first prosthetic module further comprises a second notch (53).

8. A prosthesis as claimed in claim 7, wherein the second notch and the second prosthetic side branch are aligned when the first and the second prosthetic modules are interconnected.

9. A prosthesis as claimed in any preceding claim, further comprising a third prosthetic module (64), wherein a second telescoping interconnection is established when the second and the third prosthetic modules are interconnected.

10. A prosthesis as claimed in any preceding claim, wherein the telescoping interconnection comprises one of barbs (502), two-part adhesive, one-part adhesive, and hook-and-loop fastener.

11. A prosthesis as claimed in any of claims 1 to 9, wherein the telescoping interconnection comprises an inward facing cuff on an end of the first prosthetic module engaged with an outward facing cuff on an end of the second prosthetic module.

## Patentansprüche

1. Modulare endoluminale Prothese zur Ablage in einem Körperlumen, umfassend:
ein erstes Prothesenmodul (2) und ein zweites Prothesenmodul (2);
wobei das erste Prothesenmodul einen ersten Prothesenrumpf (3) umfasst, der in einem Hauptgefäß angeordnet wird und mit einem ersten Prothesen-Seitenast (4) einstückig ist, der in einem Seitenzweiggefäß angeordnet wird; und
wobei das zweite Prothesenmodul einen zweiten Prothesenrumpf umfasst, der in einem Hauptgefäß angeordnet wird, wobei der zweite Prothesenrumpf eine erste Kerbe (11) aufweist, wobei die erste Kerbe so angeordnet ist, dass die erste Kerbe und der erste Prothesen-Seitenast miteinander ausgerichtet sind, wenn das erste und zweite Prothesenmodul miteinander verbunden sind;
worin der erste Prothesen-Seitenast in einem Seitenzweiggefäß angeordnet wird, dass von einer Seite eines Hauptgefäßes abzweigt; und
worin eine Teleskopverbindung zwischen dem ersten Prothesenrumpf und dem zweiten Prothesenrumpf hergestellt wird, wenn das erste und zweite Prothesenmodul miteinander verbunden werden, so dass die Vorrichtung angepasst werden kann, indem mehr oder weniger Überlappung zwischen dem ersten Prothesenrumpf und dem zweiten Prothesenrumpf bereitgestellt wird.

2. Prothese nach Anspruch 1, worin die erste Kerbe ein Fenster und/oder eine Bogenkante ist.

3. Prothese nach Anspruch 1 oder 2, worin die erste Kerbe (11) an einem Ende des zweiten Prothesenrumpfs vorgesehen ist.

4. Prothese nach Anspruch 1, 2 oder 3, die ferner einen zweiten Prothesen-Seitenast (42) umfasst, der mit dem zweiten Prothesenmodul einstückig ist.

5. Prothese nach Anspruch 4, worin der erste Prothesen-Seitenast und der zweite Prothesen-Seitenast allgemein auf gegenüberliegenden Seiten der Prothese angeordnet sind.

6. Prothese nach Anspruch 4, worin der erste Prothesen-Seitenast und der zweite Prothesen-Seitenast allgemein auf einer einzigen Seite der Prothese liegen.

7. Prothese nach Anspruch 4, 5 oder 6, worin das erste Prothesenmodul ferner eine zweite Kerbe (53) umfasst.

8. Prothese nach Anspruch 7, worin die zweite Kerbe und der zweite Prothesen-Seitenast miteinander ausgerichtet sind, wenn das erste und zweite Prothesenmodul miteinander verbunden sind.

9. Prothese nach einem der vorhergehenden Ansprüche, die ferner ein drittes Prothesenmodul (64) umfasst, worin eine zweite Teleskopverbindung hergestellt wird, wenn das zweite und dritte Prothesenmodul miteinander verbunden sind.

10. Prothese nach einem der vorhergehenden Ansprüche, worin die Teleskopverbindung Widerhaken (502), einen Zwei-Komponenten-Klebstoff, einen Ein-Komponenten-Klebstoff oder einen Klettverschluss umfasst.

11. Prothese nach einem der Ansprüche 1 bis 9, worin die Teleskopverbindung eine nach innen weisende Manschette an einem Ende des ersten Prothesenmoduls umfasst, die mit einer nach außen weisenden Manschette an einem Ende des zweiten Prothesenmoduls in Eingriff steht.

## Revendications

1. Prothèse endoluminale modulaire destinée à être déployée dans une lumière corporelle, comportant :
un premier module prothétique (2) et un deuxième module prothétique (2);
le premier module prothétique comportant un premier tronc prothétique (3) destiné à être placé à l'intérieur d'un vaisseau principal, d'un seul tenant avec une première ramification prothétique latérale (4) destinée à être placée dans un vaisseau sanguin latéral ; et
le deuxième module prothétique comportant un deuxième tronc prothétique destiné à être placé à l'intérieur d'un vaisseau principal, le deuxième tronc prothétique ayant une première échancrure (11), la première échancrure étant disposée de façon à ce que, quand les premier et deuxième modules prothétiques sont connectés entre eux, la première échancrure et la première ramification prothétique latérale sont alignées ;
dans laquelle la première ramification prothétique latérale est destinée à être placée dans un vaisseau sanguin latéral partant du côté d'un vaisseau principal ; et
dans laquelle une interconnexion télescopique entre le premier tronc prothétique et le deuxième tronc prothétique est établie quand les premier et deuxième modules prothétiques sont connectés entre eux, de sorte que le dispositif peut être ajusté en faisant plus ou moins se chevaucher le premier tronc prothétique et le deuxième tronc prothétique.

2. Prothèse selon la revendication 1, dans laquelle la première échancrure est soit une fenestration soit une dentelure.

3. Prothèse selon la revendication 1 ou 2, dans laquelle la première échancrure (11) est fournie à une extrémité du deuxième tronc prothétique.

4. Prothèse selon la revendication 1, 2 ou 3, comportant en outre une deuxième ramification prothétique latérale (42) d'un seul tenant avec le deuxième modèle prothétique.

5. Prothèse selon la revendication 4, dans laquelle la première ramification prothétique latérale et la deuxième ramification prothétique latérale sont en général situées de part et d'autre de la prothèse.

6. Prothèse selon la revendication 4, dans laquelle la première ramification prothétique latérale et la deuxième ramification prothétique latérale sont en général situées d'un seul côté de la prothèse.

7. Prothèse selon la revendication 4, 5 ou 6, dans laquelle le premier module prothétique comporte en outre une deuxième échancrure (53).

8. Prothèse selon la revendication 7, dans laquelle la deuxième échancrure et la deuxième ramification prothétique latérale sont alignées quand les premier et deuxième modules prothétiques sont connectés entre eux.

9. Prothèse selon l'une quelconque des revendications précédentes, comportant en outre un troisième module prothétique (64), dans lequel une deuxième interconnexion télescopique est établie quand les deuxième et troisième modules sont connectés entre eux.

10. Prothèse selon l'une quelconque des revendications précédentes, dans laquelle l'interconnexion télescopique comporte l'un des éléments suivants : des crochets (502), un adhésif en deux parties, un adhésif en une partie, et une fermeture par ruban autoagrippante.

11. Prothèse selon l'une quelconque des revendications 1 à 9, dans laquelle l'interconnexion télescopique comporte un manchon orienté vers l'intérieur sur une extrémité du premier module prothétique s'engageant dans un manchon orienté vers l'extérieur sur une extrémité du deuxième module prosthétique.
